# EUROPEAN PATENT APPLICATION

(11) **EP 4 509 602 A1**
(43) Date of publication of application: **19.02.2025**
(21) Application number: 23788100.8
(22) Date of filing: 13.03.2023
(51) Int. Cl.: C12N 15/10, C07K 1/14, C12M 1/00, C12M 3/06, C12N 7/02, G01N 1/34, G01N 33/48

(54) **METHOD FOR PRODUCING PURIFIED LIQUID HAVING INCREASED PURITY OF SEPARATION TARGET SUBSTANCE, AND PURIFICATION KIT FOR PURIFYING SEPARATION TARGET SUBSTANCE**

(30) Priority: 14.04.2022 JP 2022067069
(71) Applicant: National Institute of Advanced Industrial Science and Technology, Chiyoda-ku Tokyo 100-8921 (JP); VALQUA, Ltd., Shinagawa-ku Tokyo 141-6024 (JP)
(72) Inventor: KUNO Atsushi, Tsukuba-shi, Ibaraki 305-8560 (JP); SATO Takashi, Tsukuba-shi, Ibaraki 305-8560 (JP); WATANABE Naoki, Machida-shi, Tokyo 194-0215 (JP); SETOGUCHI Yoshihiro, Machida-shi, Tokyo 194-0215 (JP); MUTO Hirotaka, Machida-shi, Tokyo 194-0215 (JP)
(74) Representative: Dehns
(86) International application number: PCT/JP2023/009693
(87) International publication number: WO 2023/199678

(57) **Abstract**

One aspect of the present invention relates to a method for producing a purified liquid having an increased purity of a separation target substance, or a purification kit for purifying a separation target substance, and the method for producing a purified liquid having an increased purity of a substance α is a method for producing a purified liquid having an increased purity of the substance α from a sample solution containing a separation target substance (substance α) or a substance containing the substance α (substance β), the method including: (1) the following step 1 and step 2-1; or (2) the following step 1 and step 2-2 (here, the substance α in the substance β is a protein, a nucleic acid, a lipid, or a saccharide), in which a container (i) including a first chamber having an opening in an upper portion and a second chamber partitioned from the first chamber by a filter and having an opening in an upper portion is used. Step 1: a step of obtaining a container (ii) having a carrier to which the substance α or the substance β in the sample solution is adsorbed, in the first chamber. Step 2-1: a step of obtaining a purified liquid having an increased purity of the substance α by bringing a liquid capable of releasing the substance α from the carrier into contact with the carrier and releasing the substance α from the carrier, in the container (ii). Step 2-2: a step of obtaining a purified liquid having an increased purity of the substance α by bringing a liquid capable of releasing the substance α from the carrier into contact with the carrier and releasing the substance α from the carrier. (here, the filter does not allow the carrier to pass therethrough.)

## Description

### Technical Field

The present invention relates to a method for producing a purified liquid having an increased purity of a separation target substance, or a purification kit for purifying a separation target substance.

### Background Art

Chromatography for separating and purifying a target substance from a sample solution containing a plurality of substances can be classified into two types: continuous chromatography using a packed column (hereinafter referred to as column chromatography) and batch chromatography.

Column chromatography is a method in which a sample solution is brought into contact with a carrier packed in a column online, and a target substance is captured by the carrier and then released. Examples of simultaneously separating and purifying a target substance from multiple sample solutions containing, for example, proteins, nucleic acids, lipids, saccharides, viruses, and exosomes using column chromatography include a spin column using a carrier having characteristics corresponding to the target substance.

For example, in order to purify proteins, a method using a spin column that uses a carrier having adsorbability for proteins is widely used. In order to purify nucleic acids from a sample solution containing viruses, a spin column using a carrier having adsorbability for nucleic acids is widely used. In order to purify extracellular vesicles such as exosomes, in addition to the conventional ultracentrifugation method, a method using a spin column in which an antibody against an exosome marker is immobilized is common.

As a method for extracting nucleic acids from a sample solution containing nucleic acids using column chromatography, Patent Literature 1 discloses a nucleic acid extraction apparatus and a nucleic acid extraction method including: capturing means for capturing nucleic acids of a sample solution; a nucleic acid extraction instrument having a first container portion and a second container portion communicating with each other via the capturing means; means for carrying a solution into and out of the nucleic acid extraction instrument; and pressurizing and depressurizing means for pressurizing either one of the first container portion and the second container portion of the nucleic acid extraction means and depressurizing the other.

Batch chromatography is a method in which a carrier and a sample solution are mixed in a container, and then the carrier capturing a target substance and the sample solution are separated. Examples of means for separating the carrier from the sample solution include a method for centrifuging a mixture of the sample solution and the carrier and separating the carrier as a precipitate, a method for placing the mixture of the sample solution and the carrier on a filter cartridge and then centrifuging the mixture to separate the carrier as a residue on the filter, and a method for separating the carrier using magnetic beads as a carrier and using a magnet.

### Citation List

### Patent Literature

Patent Literature 1: JP 2007-306867 A

### Summary of Invention

### Technical Problem

In recent years, by a nucleic acid amplification technique such as a polymerase chain reaction method (PCR method), the encapsulated nucleic acids can be detected even from a sample solution containing an extremely small amount of viruses or exosomes. However, when the amount of viruses or exosomes is extremely small, the nucleic acid detection by the PCR method is not highly accurate, and false positives or false negatives are likely to occur. Therefore, in order to solve these problems, a method for increasing the purity of a target substance in a sample solution is required.

A sample solution containing, for example, proteins, nucleic acids, lipids, saccharides, viruses, or exosomes often contains pathogenic substances. Therefore, when a target substance is purified from such a sample solution, it is required to reduce the number of steps performed by a person as much as possible and to increase the number of steps performed by a machine from the viewpoint of safety. In recent years, automation apparatuses such as a fully automated nucleic acid extraction and amplification test system have become widespread, but the automation apparatuses may include only pipetting means for sucking and discharging a solution and means for moving the pipetting means up and down and back and forth. Therefore, in order to make the method for increasing the purity of the substance compatible with many automation apparatuses, it is important that complicated operations such as centrifugation and pressurization and depressurization are not essential.

In column chromatography, in general, since the sample solution sequentially comes into contact with the carrier, the carrier is excessively present with respect to the sample solution at the time of contact, and the target substance is easily captured. However, when the amount of solution such as the sample solution, the cleaning fluid, or the releasing solution is large, a long time is required for the operation. In addition, it is necessary to connect, for example, a tube to the downstream of the column packed with the carrier to form a flow channel. Furthermore, in order to assist the solution to pass through the column packed with the carrier, it is often necessary to pressurize and feed the solution using, for example, a liquid feeding pump or a centrifuge.

When a target substance is purified from a sample solution containing pathogenic substances by column chromatography, the entire flow channel is contaminated with the pathogenic substances, and thus the entire flow channel becomes an infectious waste. In addition, pressurization at the time of liquid feeding increases the risk that pathogenic substances leak out.

When the sample solution contains pathogenic substances, the spin column is useful in that a target substance can be quickly purified from a small amount of sample solution, and that less infectious waste is generated, but the centrifugation operation is essential in the use process. The centrifugation operation requires an expensive centrifuge, and there is a risk that the sample solution leaks and scatters due to, for example, breakage of the tube or looseness of the lid during the centrifugation operation to spread contamination. In addition, the sample solution is added to the upper portion of the spin column, and the sample solution passes through the carrier and moves to the lower portion of the container by centrifugation operation. However, the carrier cannot sufficiently capture the target substance because, for example, the target substance in the sample solution comes into contact with the carrier only once, and the carrier is clogged by impurities in the sample solution. As a result, the amount and purity of the purified target substance may not be sufficient.

The apparatus and method described in Patent Literature 1 are useful in that nucleic acids can be extracted from a sample solution having viscosity, but it is essential to increase or decrease the pressure in the container. Therefore, the risk that pathogenic substances leak out by pressurization and depressurization increases. In addition, not only the apparatus needs to include the pressurizing and depressurizing means, but also the instrument to be used must withstand the pressurization and depressurization, and the opening portion needs to be covered at the time of use. That is, the apparatus and the method described in Patent Literature 1 are not sufficiently simple in operation and cannot cope with an apparatus that does not include pressurizing and depressurizing means, and thus are not sufficient in coping with an automation apparatus.

In general, batch chromatography has an advantage of being able to perform separation quickly as separation can be performed at once even when a large amount of sample solution is used. On the other hand, since the total amount of the sample solution comes into contact with the carrier at once, the sample solution is excessively present with respect to the carrier, and when the binding properties between the target substance and the carrier are not so strong, the target substance may not be sufficiently captured. In addition, the centrifugation operation or use of magnetic beads is essential, but there is a risk as described above in the centrifugation operation, and there is a problem that the magnetic beads are expensive and it is difficult to recover the magnetic beads when, for example, the concentration of the target substance in the sample solution is low.

One aspect of the present invention provides a method for producing a purified liquid having an increased purity of a separation target substance from a sample solution containing the separation target substance or a substance containing the separation target substance by a simple operation, and a purification kit.

### Solution to Problem

The present inventors have intensively studied to solve the above problems. As a result, the present inventors have found that the above problem can be solved by having the following configuration, and have completed the present invention.

Aspects of the present invention relate to, for example, the following [1] to [12].
[1] A method for producing a purified liquid having an increased purity of a substance α from a sample solution containing a separation target substance (substance α) or a substance containing the substance α (substance β), the method including:
   (1) step 1 and step 2-1 which will be described below; or
   (2) step 1 and step 2-2 which will be described below (here, the substance α in the substance β is a protein, a nucleic acid, a lipid, or a saccharide),
      in which a container (i) including a first chamber having an opening in an upper portion and a second chamber partitioned from the first chamber by a filter and having an opening in an upper portion is used.
         Step 1: a step of obtaining a container (ii) having a carrier to which the substance α or the substance β in the sample solution is adsorbed, in the first chamber.
         Step 2-1: a step of obtaining a purified liquid having an increased purity of the substance α by bringing a liquid capable of releasing the substance α from the carrier into contact with the carrier and releasing the substance α from the carrier, in the container (ii).
         Step 2-2: a step of obtaining a purified liquid having an increased purity of the substance α by bringing a liquid capable of releasing the substance α from the carrier into contact with the carrier and releasing the substance α from the carrier.
      (here, the filter does not allow the carrier to pass therethrough.)
[2] The method for producing a purified liquid according to [1], in which the step 2-1 or the step 2-2 is performed by a pipetting operation.
[3] The method for producing a purified liquid according to [1] or [2], in which the container (ii) is obtained by any one of the following steps a to d.
   Step a: a step of adsorbing the substance α or the substance β to the carrier by putting the sample solution into the second chamber and diffusing the substance α and the substance β in the sample solution, after a carrier capable of adsorbing the substance α or the substance β is put into the first chamber (here, the filter allows the substance α and the substance β to pass therethrough).
   Step b: a step of adsorbing the substance α or the substance β to the carrier by putting the sample solution into the first chamber and diffusing the substance α and the substance β in the sample solution, after a carrier capable of adsorbing the substance α or the substance β is put into the first chamber.
   Step c: a step of adsorbing a substance containing the substance α or the substance β to the carrier by providing the first chamber having a carrier capable of adsorbing the substance α or the substance β and the second chamber, and diffusing the substance α and the substance β in the sample solution, in a container (iii), after the sample solution is put into the container (iii) having an opening in the upper portion (here, the substance α and the substance β are allowed to pass through the filter).
   Step d: a step of putting a carrier to which the substance α or the substance β is adsorbed into the first chamber after obtaining a carrier to which a substance containing the substance α or the substance β is adsorbed, by mixing the sample solution with a carrier capable of adsorbing the substance α or the substance β, and diffusing the substance α and the substance β in the sample solution.
[4] The method for producing a purified liquid according to [3], in which the method for diffusing the substance α and the substance β in the sample solution is a method for moving a liquid surface of the sample solution up and down.
[5] The method for producing a purified liquid according to [3] or [4], in which the method for diffusing the substance α and the substance β in the sample solution is performed by a pipetting operation.
[6] The method for producing a purified liquid according to any one of [3] to [5], further including: a step of removing a liquid present in the container (ii) after any one of the steps a to d, after any one of the steps a to d and before bringing the liquid capable of releasing the substance α from the carrier into contact with the carrier.
[7] The method for producing a purified liquid according to [6], in which the step of removing the liquid present in the container (ii) after any one of the steps a to d is performed by the pipetting operation.
[8] The method for producing a purified liquid according to [6] or [7], in which
   the method for diffusing the substance α and the substance β in the sample solution,
   the step of removing the liquid present in the container (ii) after any one of the steps a to d, and
   any one of the step 2-1 and the step 2-2, are performed by the pipetting operation.
[9] The method for producing a purified liquid according to any one of [1] to [8], in which the substance α is a nucleic acid or a protein.
[10] The method for producing a purified liquid according to any one of [1] to [9], in which the substance β is a virus or an exosome.
[11] The method for producing a purified liquid according to any one of [1] to [9], wherein the substance α is an exosome.
[12] A purification kit which is a purification kit for purifying a substance α from a sample solution containing a separation target substance (substance α) or a substance containing the substance α (substance β), the purification kit including:
   a container, a carrier capable of adsorbing the substance α or the substance β, and a filter that does not allow the carrier to pass through, in which
   the filter is used to partition the container into a first chamber and a second chamber each having an opening in an upper portion, and the first chamber has the carrier.

### Advantageous Effects of Invention

According to one aspect of the present invention, it is possible to provide a method for producing a purified liquid having an increased purity of a separation target substance from a sample solution containing a separation target substance or a substance containing the separation target substance by a simple operation, and a purification kit.

### Brief Description of Drawings

Fig. 1 shows an amplification curve in quantitative PCR for M gene of influenza A virus in Example 2.
Fig. 2 shows an amplification curve in quantitative PCR for miR181a in Example 3.
Fig. 3 shows an amplification curve in quantitative PCR for M gene of influenza A virus in Example 4.
Fig. 4 shows an amplification curve in quantitative PCR for the M gene of influenza A virus in Example 5.
Fig. 5 shows an amplification curve in quantitative PCR for miR181a in Example 6.
Fig. 6 shows an amplification curve in quantitative PCR for miR181a in Example 7.
Fig. 7 shows an amplification curve in quantitative PCR for miR181a in Example 8.
Fig. 8 relates to Example 9. Fig. 8(a) is a diagram showing an outline of an experiment of Example 9. Fig. 8(b) is a diagram showing the results of detecting CD9 by Western blot in Example 9.

### Description of Embodiments

Next, the present invention will be specifically described.

The expression "A to B" in the numerical range means A or more and B or less unless otherwise specified. In addition, % means mass%.

### <<Method for Producing Purified Liquid>>

According to one aspect of the present invention, a method for producing a purified liquid having an increased purity of a substance α from a sample solution containing a separation target substance (hereinafter also referred to as "substance α") or a substance containing the separation target substance (hereinafter also referred to as "substance β"), is a method including the following steps 1 and 2-1 in which a container (i) having a first chamber having an opening in an upper portion and a second chamber partitioned from the first chamber by a filter and having an opening in an upper portion is used.

Step 1: a step of obtaining a container (ii) having a carrier to which the substance α or the substance β in the sample solution is adsorbed, in the first chamber.

Step 2-1: a step of obtaining a purified liquid having an increased purity of the substance α by bringing a liquid capable of releasing the substance α from the carrier into contact with the carrier and releasing the substance α from the carrier, in the container (ii).
(here, the filter does not allow the carrier to pass therethrough.)

Hereinafter, this production method is also referred to as "production method (X)-1".

According to one aspect of the present invention, a method for producing a purified liquid having an increased purity of a substance α from a sample solution containing a separation target substance (hereinafter also referred to as "substance α") or a substance containing the separation target substance (hereinafter also referred to as "substance β", and here, the substance α in the substance β is a protein, a nucleic acid, a lipid, or a saccharide), is a method for producing a purified liquid including the following steps 1 and 2-2 in which a container (i) having a first chamber having an opening in an upper portion and a second chamber partitioned from the first chamber by a filter and having an opening in an upper portion is used.

Step 1: a step of obtaining a container (ii) having a carrier to which the substance α or the substance β in the sample solution is adsorbed, in the first chamber.

Step 2-2: a step of obtaining a purified liquid having an increased purity of the substance α by bringing a liquid capable of releasing the substance α from the carrier into contact with the carrier and releasing the substance α from the carrier.
(here, the filter does not allow the carrier to pass therethrough.)

Hereinafter, this production method is also referred to as "production method (X)-2".

The "production method (X)-1" and the "production method (X)-2" are hereinafter also collectively referred to as "production method (X)".

### [Substance α and Substance β]

The substance α is a substance produced by the production method (X) and having an increased purity in the purified liquid.

The substance α and the substance β contained in the sample solution may be one type or two or more types. In addition, the substance α contained in the purified liquid may be one type or two or more types.

In the "production method (X)-1", the substance α is not particularly limited, and examples thereof include proteins, nucleic acids, lipids, and saccharides, which may be labeled with, for example, a fluorescent dye, biotin, a reporter enzyme, or a radioisotope. In the "production method (X)-1", the substance α is preferably a nucleic acid or a protein.

In the "production method (X)-2", the substance α is not particularly limited, and examples thereof include, in addition to proteins, nucleic acids, lipids, and saccharides, viruses; extracellular vesicles such as exosomes, microvesicles, and apoptotic vesicles; drug delivery system (DDS) preparations such as liposomes and lipid nano particles (LNPs), and these may be labeled with, for example, a fluorescent dye, biotin, a reporter enzyme, or a radioisotope. In the "production method (X)-2", the substance α is preferably a nucleic acid, a protein, or an exosome.

The protein is not particularly limited, and examples thereof include simple proteins such as albumin, globulin, keratin, collagen, and fibroin; and complex proteins such as glycoproteins, phosphoproteins, chromoproteins, and nucleoproteins. Among these, simple proteins are preferable.

The protein may be an antibody, a contractile protein, an enzyme, a hormonal protein, a structural protein, a storage protein, or a transport protein, and is preferably an antibody or a transport protein.

The nucleic acid is not particularly limited, and examples thereof include DNA, RNA, microRNA, and mRNA.

The lipid is not particularly limited, and is, for example, a simple lipid such as glyceride, sterol ester, wax, or ceramide; complex lipids such as phospholipids and glycolipids; and derived lipids such as fatty acids, terpenoids, steroids, and carotenoids.

The saccharide is not particularly limited, and examples thereof include monosaccharides such as glucose and fructose; disaccharides such as maltose, sucrose, and lactose; polysaccharides such as starch, cellulose, and glycogen; and glycans that are added to proteins and lipids in eukaryotes and exist as components of the complex.

The form of the substance β is not particularly limited as long as the substance β contains the substance α, and the substance β is preferably a substance in which the substance α is contained inside an encapsulated substance.

The encapsulated substance is, for example, a capsule-like substance that encapsulates at least a part of the substance α.

In the "production method (X)-2", the substance β exists only when the substance α is a protein, a nucleic acid, a lipid, or a saccharide. That is, in the "production method (X)-2", the substance α in the substance β is a protein, a nucleic acid, a lipid, or a saccharide.

Examples of the substance in which the substance α is contained in the encapsulated substance include viruses; extracellular vesicles such as exosomes, microvesicles, and apoptotic vesicles; and drug delivery system (DDS) formulations such as liposomes and lipid nano particle (LNP). The substance β is preferably a virus or an exosome.

In the virus, nucleic acids are contained in an encapsulated substance (for example, capsid and envelope) composed of at least one selected from proteins, lipids, and sugars.

Examples of such viruses include enveloped viruses such as viruses from the Flaviviridae family (for example, Dengue virus, Hepatitis C virus, and Japanese encephalitis virus), the Orthomyxoviridae family (for example, influenza virus), the Coronaviridae family (for example, SARS coronavirus, SARS-CoV-2, and MERS coronavirus), the Togaviridae family (for example, Rubella virus), the Paramyxoviridae family (for example, Measles virus and Mumps virus), and the Hepadnaviridae family (for example, Hepatitis B virus); and non-enveloped viruses such as viruses from the Caliciviridae family (for example, norovirus), the Papillomaviridae family (for example, Papillomavirus), the Reoviridae family, (for example, Reovirus and Rotavirus), and the Picornaviridae family (for example, Hepatitis A virus).

Among these, enveloped viruses are preferable, viruses from the Orthomyxoviridae family are more preferable, and influenza viruses are still more preferable.

In the extracellular vesicle, at least one selected from nucleic acids, proteins, lipids, and sugars is contained in an encapsulated substance composed of at least one selected from proteins, lipids, and sugars.

Examples of the extracellular vesicles include exosomes, microvesicles, and apoptotic vesicles, and among these, exosomes are preferable.

In the liposome, at least one selected from nucleic acids, proteins, lipids, and sugars is contained in an encapsulated substance (for example, a lipid bilayer) composed of lipids.

In the LNP, at least one selected from nucleic acids, proteins, lipids, and sugars is contained in an encapsulated substance (for example, a lipid monolayer or a lipid bilayer composed of, for example, ionized lipids, helper lipids, or PEG lipids) composed of lipids.

### [Sample Solution]

The sample solution is not particularly limited as long as the sample solution is a liquid that may contain the substance α or the substance β. Examples of the sample solution include a biological sample, a food, an environmental sample, a microorganism or a cell sample, and a liquid obtained by diluting these with a diluent.

Examples of the biological sample include animal and plant tissues, body fluids, excretions, and swab fluids, cleaning fluids, and cultures thereof. More specific examples include blood, plasma, serum, blood culture solution, urine, saliva, amniotic fluid, pus, cerebrospinal fluid, pleural effusion, pharyngeal swab fluid, nasal swab fluid, nasopharyngeal swab fluid, nasal discharge, sputum, rectal swab fluid, tissue section, skin, vomit, feces, myringotomy fluid, alveolar lavage fluid, gastric lavage fluid, intestinal lavage fluid, cervical swab fluid, urethral abrasion, organ extract, tissue extract, cell disrupted fluid, and isolated culture colonies.

According to one aspect of the present invention, since it is possible to increase the purity of the substance α even when a biological sample containing a large amount of impurities is targeted, the biological sample is preferably blood, urine, saliva, a pharyngeal swab fluid, a nasal swab fluid, a nasopharyngeal swab fluid, nasal discharge, or sputum, and among these, a sample derived from a mammal, particularly, derived from a human is more preferable.

Examples of the food include water, alcoholic beverages, soft drinks, processed foods, vegetables, livestock products, marine products, eggs, dairy products, raw meat, raw fish, and side dishes. When a food is used as a sample, not only a part or all of the food can be used, but also a product obtained by wiping the surface of the food can be used. Furthermore, a product obtained by wiping a food contact portion or a human contact portion of a cooking utensil, for example, or a cleaning fluid obtained by cleaning the food contact portion or the human contact portion can also be used as the sample. For a sample containing a large amount of liquid components, a sample obtained by performing, for example, drying, ultrafiltration, or distillation to remove a part or all of the liquid components may be used as necessary.

Examples of the environmental sample include water, ice, and soil. Examples of the water herein include water collected from various sources, such as tap water, seawater, rivers, waterfalls, lakes, and ponds. In addition, a sample obtained by wiping, for example, human contact portions such as door knobs, facility wall surfaces, floor surfaces, equipment and fixtures, or toilets, or a cleaning fluid obtained by cleaning these can also be used as the sample. For a sample containing a large amount of liquid components, a sample obtained by performing, for example, drying, ultrafiltration, or distillation to remove a part or all of the liquid components may be used as necessary.

The microorganisms or cell samples may be, for example, transformants into which recombinant vectors for expressing proteins of interest is introduced, or hybridomas in which a plurality of cells are fused, in addition to naturally derived microorganisms or cells. Further, in addition to the microorganisms or cells themselves, a microorganism or cell lysates and culture supernatants are also included.

Examples of the microorganism or cell include cultured cells such as Escherichia coli, yeast, plant cells, insect cells, and animal cells, and Escherichia coli and animal cells are preferable.

In the "production method (X)-2", the microorganism or cell sample is preferably an animal cell, and more preferably a culture supernatant of an animal cell. The animal cell may be derived from any animal, but is preferably derived from a mammal, more preferably derived from a human, bovine, canine, feline, porcine, minipig, rabbit, hamster, rat, or mouse cell, and still more preferably derived from a human cell. The animal cells may be primary cells, established cells, or cells derived from, for example, iPS cells or ES cells.

The diluent is not particularly limited, and examples thereof include water and a buffer solution. As the buffer solution, for example, a buffer solution usually used in a biochemical test can be used, and examples thereof include a Tris buffer solution and a phosphate buffer solution. The diluent contains, for example, a salt such as sodium chloride; surfactants such as SDS; metal chelating agents such as EDTA; preservatives such as sodium azide can be appropriately added.

In the "production method (X)-2", the diluent may be, for example, a culture medium for culturing a microorganism or a cell. In the "production method (X)-2", the diluent is preferably a culture medium for cell culture, and more preferably a culture medium containing no animal-derived serum.

The method for collecting the sample solution is not particularly limited, and a known method can be used according to the type and purpose of the sample solution. Examples of the collection method include a collection method using collection tools such as cotton swabs, swabs, inoculation loops, pipettes, spatulas, scoops, and syringes.

The amount of the sample solution used in the production method (X) is not particularly limited, and may be determined according to, for example, the concentration of the substance α or the substance β expected to be contained in the sample solution, the volume of the container.

For example, when a container having a volume of about 15 mL is used, the amount of the sample solution is preferably 0.5 mL to 14 mL, and more preferably 1 mL to 12 mL, and when a container having a volume of about 2 mL is used, the amount of the sample solution is preferably 100 µL to 1.5 mL, and more preferably 500 µL to 1.2 mL.

The amount of the sample solution used in the "production method (X)-2" is, for example, preferably 10 mL to 50 mL, and more preferably 20 mL to 40 mL when a container having a volume of about 50 mL (for example, a cell culture flask having a culture area of 25 cm²) is used, and the amount of the sample solution is preferably 30 mL to 150 mL, and more preferably 60 mL to 120 mL when a container having a volume of about 150 mL (for example, a cell culture flask having a culture area of 75 cm²) is used.

When the sample solution is a protein suspension, the protein concentration of the sample solution is preferably 0.1 to 10,000 µg/mL, and more preferably 1 to 1000 µg/mL.

When the sample solution is a virus suspension, the virus concentration (titer) of the sample solution is a concentration at which viral nucleic acid can be detected by, for example, PCR after concentration, and is preferably 200 TCID₅₀/mL or more, and more preferably 2000 TCID₅₀/mL. In order to improve the storage stability of the virus, the sample solution may contain preferably 0.1 to 100,000 µg/mL, and more preferably 1 to 10,000 µg/mL of protein (for example, BSA).

When the sample solution is an exosome suspension, the exosome concentration of the sample solution is preferably 0.1 to 10.0 µg/mL, and more preferably 1.0 to 10.0 µg/mL.

When the sample solution used in the "production method (X)-2" is a cell culture supernatant, since there is a tendency that the amount of components derived from dead cells is small, a cell culture supernatant obtained after culturing cells for 12 hours to 7 days is preferable, and a cell culture supernatant obtained after culturing cells for 24 hours to 5 days is more preferable. The cell culture supernatant may be used after being diluted, for example, 2 to 5 times with the medium used for cell culture.

### [Purified Liquid]

The purified liquid produced by the production method (X) can be used for various applications because the amount of impurities is reduced and the purity of the substance α is increased as compared with the sample solution. For example, when the purified liquid is used in a method for detecting a trace substance, such as a PCR method, the trace substance is easily detected with higher accuracy. In addition, since the production method (X) is also suitable for increasing the purity of an expensive substance α having activity such as an antibody, the purified liquid can be used for the production of biopharmaceuticals such as antibody drugs, vaccines, and protein preparations.

The purified liquid produced by the production method (X) is a purified liquid having an increased purity of the substance α from the sample solution containing the substance α or the substance β. The increased purity of the substance α means that the ratio of the substance α to substances other than the substance α increases. According to the production method (X), for example, when the purity of the substance α in the sample solution is 100%, a purified liquid having a purity of preferably 400% or more, and more preferably 500% or more can be obtained.

### [Container]

In the "production method (X)-1", the container is a vessel for performing a series of steps from a sample solution containing the substance α or the substance β to production of a purified liquid having an increased purity of the substance α, and is a vessel in which at least a first chamber and a second chamber are formed.

In the "production method (X)-2", the container is a vessel used for producing a purified liquid having an increased purity of the substance α from a sample solution containing the substance α or the substance β, and is a vessel in which at least a first chamber and a second chamber are formed.

In addition, for example, a third chamber other than the first chamber and the second chamber may be formed in the container as necessary.

The shape of the container is not particularly limited, but a bottomed tubular container such as a test tube, a microtube, a beaker, a flask, or a dish (for example, a petri dish) is preferable because it is easy to perform a pipetting operation. It is preferable that the container has an opening in an upper portion in order to input a sample solution or a carrier, and the opening in the upper portion is closable in order to prevent contamination.

In the "production method (X)-1", the size of the container is not particularly limited, and may be appropriately selected according to the type and amount of the sample solution. The volume of the container is preferably 0.2 mL to 100 mL, more preferably 1 mL to 60 mL, and still more preferably 5 mL to 50 mL because the pipetting operation is easily performed.

In the "production method (X)-2", the size of the container is not particularly limited, and may be appropriately selected according to the type and amount of the sample solution. The volume of the container is preferably 0.2 mL to 500 mL, more preferably 1 mL to 200 mL, and still more preferably 5 mL to 50 mL because the pipetting operation is easily performed.

The material constituting the container may be appropriately selected according to the type of the sample solution, and is not particularly limited as long as the material has non-adsorbability for the substance α or the substance β and does not have reactivity with the sample solution, and examples thereof include synthetic resins such as polystyrene, polycarbonate, polyethylene, polypropylene, polyethylene terephthalate, polyetherimide, polyimide, ABS resin, polyvinyl chloride, polyvinylidene chloride, and fluororesin; glass; and metal such as stainless steel. These may be used singly, or in combination of two or more types thereof.

The container is preferably a transparent or translucent container. Furthermore, the filter is preferably more transparent than a filter which will be described later. When the container is transparent or translucent, for example, the position of the pipette tip, the state where, for example, the sample solution is sucked and discharged, and the state where the sample solution and the carrier are stirred are easily visually recognized from the outside of the container. Therefore, it becomes easier to cope with the automation apparatus.

The container having a first chamber having an opening in an upper portion and a second chamber partitioned from the first chamber by a filter and having an opening in an upper portion is also referred to as "container (i)".

A container having a carrier to which the substance α or the substance β in the sample solution is adsorbed in the first chamber is also referred to as "container (ii)".

A container having an opening in the upper portion is also referred to as "container (iii)".

The container (i) becomes the container (ii) when the first chamber has a carrier to which the substance α or the substance β in the sample solution is adsorbed.

The container (iii) becomes the container (ii) when a first chamber having a carrier capable of adsorbing the substance α or the substance β and having an opening in an upper portion and a second chamber partitioned from the first chamber by a filter and having an opening in an upper portion are provided in the container (iii).

### [First Chamber]

The first chamber is a space in the container (i) having an opening in an upper portion for having a carrier to which the substance α or the substance β in the sample solution is adsorbed.

The shape of the first chamber is not particularly limited as long as the first chamber has an opening in the upper portion, but a shape capable of performing a removal step and/or a cleaning step which will be described later is preferable.

The opening may be at least a part of the upper portion of the first chamber, but preferably the entire upper portion of the first chamber is opened. The shape and size of the opening are also not particularly limited, but a shape and size into which a pipette tip can be inserted are preferable.

It is preferable that the opening is located above the maximum height of the liquid surface of the sample solution, and it is preferable that the carrier and the sample solution do not leak from such an opening.

The volume of the first chamber is not particularly limited as long as the carrier to which the substance α or the substance β in the sample solution is adsorbed can be contained in the first chamber, but it is preferable that the volume is large enough to perform the removal step and/or cleaning step which will be described below.

The amount of the carrier to be contained is not particularly limited, but the volume of the carrier with respect to the volume of the first chamber is preferably 1/10 to 1/500, more preferably 1/50 to 1/400, and even more preferably 1/100 to 1/200. Within the above range, a space in which the carrier can be dispersed is sufficiently secured in the first chamber, and thus the substance α or the substance β is easily adsorbed to the carrier.

The method for containing the carrier to which the substance α or the substance β in the sample solution is adsorbed into the first chamber is not particularly limited as long as the carrier is contained to be immersed in the sample solution.

### [Second Chamber]

The second chamber is a space in the container (i) that is partitioned from the first chamber by a filter that does not allow the carrier to pass therethrough and has an opening in an upper portion. The filter will be described later.

The shape and the volume of the second chamber are not particularly limited as long as the second chamber has an opening in the upper portion, but a shape and a volume capable of performing a removal step and/or a cleaning step which will be described later are preferable.

The opening may be at least a part of the upper portion of the second chamber, but preferably the entire upper portion of the second chamber is opened. The shape and size of the opening are also not particularly limited, but a shape and size into which a pipette can be inserted are preferable.

It is preferable that the opening is located above the maximum height of the liquid surface of the sample solution, and it is preferable that the sample solution do not leak from such an opening.

In the partition between the first chamber and the second chamber, at least a part of the region may be composed of the filter, a part of the region may be composed of the filter, and the remaining region may be composed of a material other than the filter, but it is preferable that all the regions are composed of the filter. The position of the region composed of the filter is not particularly limited as long as it is a position to be immersed in the sample solution, and is, for example, a side portion or a lower portion of the first chamber.

The material other than the filter is not particularly limited as long as the material has non-adsorbability for the substance α or the substance β and does not have reactivity with the sample solution, and examples thereof include synthetic resins such as polystyrene, polycarbonate, polyethylene, polypropylene, polyethylene terephthalate, polyetherimide, polyimide, ABS resin, polyvinyl chloride, polyvinylidene chloride, and fluororesin; glass; and metal such as stainless steel. These may be used singly, or in combination of two or more types thereof.

When the first chamber and/or the second chamber are formed, a spacer may be inserted into the first chamber and/or the second chamber for the purpose of easily performing a pipetting operation in each chamber, particularly a process of, for example, facilitating insertion of a pipette tip.

The shape and size of the spacer are not particularly limited as long as the spacer can be inserted into the first chamber and the second chamber, but for example, a spiral spacer can be preferably used.

The material constituting the spacer is not particularly limited as long as the material has non-adsorbability for the substance α or the substance β and does not have reactivity with the sample solution, and examples thereof include synthetic resins such as polystyrene, polycarbonate, polyethylene, polypropylene, polyethylene terephthalate, polyetherimide, polyimide, ABS resin, polyvinyl chloride, polyvinylidene chloride, and fluororesin; glass; and metal such as stainless steel. These may be used singly, or in combination of two or more types thereof.

Since each of the first chamber and the second chamber has an opening in an upper portion, all steps of the production method (X) can be performed by a pipetting operation. Therefore, the production method (X) can also cope with an automation apparatus that only includes pipetting means for sucking and discharging a solution and means for moving the pipetting means up and down and back and forth. Then, since the number of steps performed by a person is reduced and the number of steps performed by a machine is increased, the safety of work can be enhanced.

Further, even when the entire region of the partition between the first chamber and the second chamber is formed of the filter, a sufficient space can be secured at the pipette tip insertion position by spacer insertion, and thus, the production method (X) can cope with an automation apparatus.

### [Filter]

The filter is a partition for separating the first chamber and the second chamber, and is a filter that does not allow the carrier to pass therethrough, specifically, a filter having a hole with a size that does not allow the carrier to pass therethrough.

The pore diameter of the filter is not particularly limited as long as the pore diameter does not allow the carrier to pass, that is, the pore diameter is smaller than the size of the carrier. The pore diameter of the filter can be appropriately selected in a range of about 100 nm or more and less than 2 mm corresponding to the size of the carrier to be used, and is preferably 100 nm to 20 µm.

In general batch chromatography, it is considered that the amount ratio of the carrier to the sample solution is always constant while the sample solution and the carrier are in contact with each other as one of factors that the target substance cannot be sufficiently captured. On the other hand, although the production method (X) is a type of batch chromatography, the carrier cannot pass through the filter and thus remains in the first chamber, but the solvent of the sample solution passes through the filter and moves back and forth between the first chamber and the second chamber, and the substance α and the substance β in the sample solution can pass through the filter and move back and forth between the first chamber and the second chamber according to the pore diameter of the filter. Therefore, the amount ratio of the carrier to the sample solution changes while the sample solution is in contact with the carrier. Therefore, the substance α or the substance β can be sufficiently adsorbed to the carrier.

In steps a and c which will be described later, a filter (hereinafter also referred to as "filter A") which does not pass through the carrier and through which the substance α and the substance β can pass is used. For example, the pore diameter of the filter A is not limited as long as the filter A does not allow the carrier to pass therethrough and the substances α and β can pass through the filter A.

For example, when a sample solution containing viruses or exosomes is used, in general, the diameter of the viruses is about 100 to 200 nm, the diameter of the exosomes is about 20 to 200 nm, and the average particle diameter of the carrier is preferably 0.5 µm to 2 mm. Therefore, the pore diameter of the filter A can be appropriately selected in the range of about 200 nm to 2 mm corresponding to these diameters, but is preferably 250 nm to 20 µm.

In addition, for example, when a sample solution containing a protein is used, in general, the diameter of the protein is about 1 to 100 nm, and the average particle diameter of the carrier is preferably 0.5 µm to 2 mm. Therefore, the pore diameter of the filter A can be appropriately selected in the range of about 100 nm to 2 mm, corresponding to these diameters, but is preferably 250 nm to 20 µm.

In the following step a or step c in the case of providing the first chamber and the second chamber in the container (iii) by the method (II), it is preferable to further use a filter that does not allow impurities in the sample solution to pass as the filter A. Using such a filter in the step a and the step c in the case of providing the first chamber and the second chamber in the container (iii) by the method (II), the impurities remain in the second chamber, and the removal step of removing the liquid present in the container (ii) after the step a or the step c is performed, particularly when the removal step is performed in the second chamber, most of the impurities in the sample solution can be removed in the removal step, and the carrier to which the substance α or the substance β is adsorbed and the impurities can be sufficiently separated, and thus a purified liquid having higher purity of the substance α can be easily produced.

In the step b and the step d which will be described later, the filter is not particularly limited as long as the filter that does not allow the carrier to pass therethrough is used, but when the substance α (for example, nucleic acid) is to be released into the liquid from the substance β (for example, encapsulated substance containing nucleic acids) adsorbed to the carrier, the filter may be a filter that allows the substance α to pass therethrough and does not allow the carrier and the substance β to pass therethrough (hereinafter also referred to as "filter B"). For example, the pore diameter of the filter B is not limited as long as the filter B allows the substance α to pass therethrough and does not allow the carrier and the substance β to pass therethrough.

For example, when a sample solution containing viruses or exosomes is used, in general, the diameter of the viruses is about 100 to 200 nm, the diameter of the exosomes is about 20 to 200 nm, and the average particle diameter of the carrier is preferably 0.5 µm to 2 mm. Therefore, the pore diameter of the filter B can be appropriately selected in the range of about 1 nm to 2 mm according to these diameters, but is preferably 1 nm to 200 nm.

The form of the filter is not particularly limited, and examples thereof include a porous membrane having a continuous pore structure, or a woven fabric or a nonwoven fabric composed of fibers or ultrafine fibers, but a porous membrane or a nonwoven fabric is preferable, and a nonwoven fabric is more preferable. The ultrafine fiber is a fiber having a diameter of 5 µm or less, and preferably a fiber having a diameter of about 1 µm.

The material constituting the filter is not particularly limited, but a material that hardly adsorbs the substance α and the substance β is preferable.

The filter is preferably a filter having hydrophilicity from the viewpoint that, for example, the substance α and the substance β are hardly adsorbed. Examples of the filter having hydrophilicity include a filter made of a hydrophilic material and a filter obtained by hydrophilizing a filter made of a material having insufficient hydrophilicity.

Examples of the material of the filter include thermoplastic resins such as polyolefin (for example, polyethylene and polypropylene), polystyrene, ABS resin, AS resin, EVA resin, polymethylpentene, polyvinyl chloride, polyvinylidene chloride, poly(meth)acrylic acid ester, polyvinyl acetate, polyamide, polyimide, polycarbonate, polyethylene terephthalate, polybutylene terephthalate, polyacetal, polyarylate, polysulfone, and fluororesin; biodegradable resins such as polylactic acid, polyhydroxybutyrate, modified starch resin, polycaprolactone, polybutylene succinate, polybutylene adipate terephthalate, polybutylene succinate terephthalate, and polyethylene succinate; thermosetting resins such as phenol resin, urea resin, melamine resin, unsaturated polyester resin, diallyl phthalate resin, epoxy resin, epoxy(meth)acrylate resin, silicon resin, (meth)acrylic urethane resin, and urethane resin; elastomers such as silicone resins, polystyrene elastomers, polyethylene elastomers, polypropylene elastomers, polyurethane elastomers, and fluoroelastomers (for example, FKM and FFKM); natural materials such as pulp, hemp, cellulose, kenaf, chitin, chitosan, and cotton; inorganic material such as glass, silica, or metal. These materials may be used singly or in combination of two or more types thereof.

Among these materials, a fluororesin and a fluoroelastomer are more preferable as a material constituting the filter, and a filter composed of a hydrophilized fluororesin or a hydrophilized fluoroelastomer (particularly, hydrophilized FKM) is still more preferable.

The fluororesin is not particularly limited, and examples thereof include polytetrafluoroethylene (PTFE), tetrafluoroethylene-perfluoroalkyl vinyl ether copolymer (PFA), tetrafluoroethylene-hexafluoropropylene copolymer (FEP), ethylene-tetrafluoroethylene copolymer (ETFE), tetrafluoroethylene-hexafluoropropylene-perfluoroalkyl vinyl ether copolymer (EPE), fluoroethylene-vinyl ether copolymer (FEVE), poly(chlorotrifluoroethylene) (PCTFE), ethylene-chlorotrifluoroethylene-copolymer (ECTFE), polyvinylidene fluoride (PVDF), polyvinyl fluoride (PVF), vinylidene fluoride-hexafluoropropylene copolymer (VDF-HFP copolymer), and vinylidene fluoride-hexafluoropropylene-tetrafluoroethylene copolymer (VDF-HFP-TFE copolymer). Among these, PTFE and PVDF are preferable from the viewpoint of further exhibiting the effects of the present invention and further, for example, excellent chemical resistance.

The fluoroelastomer is not particularly limited, but examples thereof include perfluoro(alkyl vinyl ether), perfluoro(alkoxyalkyl vinyl ether), vinylidene fluoride-hexafluoropropylene-based polymer, vinylidene fluoride-hexafluoropropylene-tetrafluoroethylene-based polymer, tetrafluoroethylene-propylene-based polymer, vinylidene fluoride-propylene-tetrafluoroethylene-based polymer, ethylene-tetrafluoroethylene-perfluoromethyl vinyl ether-based polymer, vinylidene fluoride-tetrafluoroethylene-perfluoromethyl vinyl ether-based polymer, and vinylidene fluoride-perfluoromethyl vinyl ether-based polymer. Among these, a ternary FKM is preferable from the viewpoint of, for example, excellent heat resistance or chemical resistance, and vinylidene fluoride-hexafluoropropylene-tetrafluoroethylene-based polymer is more preferable.

The hydrophilization treatment preferably includes a step of coating a filter with a compound having a hydrophilic group (hereinafter also referred to as "hydrophilic compound"), and more preferably includes a step of immersing the filter in a solution of a hydrophilic compound, coating the filter with the hydrophilic compound, and then crosslinking the hydrophilic compound. Specific examples of such a step include steps described in WO 2014/021167 and JP 4-075051 B.

In the "production method (X)-2", the hydrophilization treatment may be repeated a plurality of times. The filter may be immersed in a solution of a hydrophilic compound, coated with the hydrophilic compound, then crosslinked with the hydrophilic compound, and then further the filter may be immersed in a solution of a hydrophilic compound that is the same as or different from the hydrophilic compound which was already used, and the filter may be coated with the hydrophilic compound.

The hydrophilic compound is not particularly limited as long as the effect of the present invention is not impaired, and a compound having any shape such as a linear shape, a branched shape, or a dendrimer shape can be used.

Examples of the hydrophilic compound include a hydroxyl group-containing compound, a carboxylic acid group-containing compound, a sulfonic acid group-containing compound, an ether group-containing compound, an epoxy group-containing compound, an amino group-containing compound, an amide group-containing compound, and a phosphorylcholine group-containing compound.

The hydrophilic compound may be used singly or in combination of two or more types thereof.

The hydroxyl group-containing compound is not particularly limited, and examples thereof include polyvinyl alcohol (PVA) and modified products thereof (for example, ethylene oxide group-modified PVA, carboxyl group-modified PVA, sulfonic acid group-modified PVA, and quaternary ammonium-modified PVA); polysaccharides such as agarose, dextran, chitosan, cellulose, and heparin, and derivatives thereof; collagen; gelatin; copolymer of vinyl alcohol and vinyl group-containing monomer (for example, vinyl alcohol-vinyl acetate copolymer, ethylene-vinyl alcohol copolymer, vinyl alcohol-polyvinyl pyrrolidone copolymer); polyols such as (meth)acrylic polyol, fluorine-containing polyol, polyoxyalkylene (for example, polyethylene glycol, copolymer of polyethylene glycol and polypropylene glycol (for example, Pluronic F108 and Pluronic F127 (both manufactured by Sigma-Aldrich Co. LLC)), polyester polyol, and diethylene glycol; and hydroxyl group-containing (meth)acrylic compounds such as poly(2-hydroxyethyl(meth)acrylate), 1-hydroxypropane-2-yl=(meth)acrylate, 2-hydroxypropane-1-yl=(meth)acrylate (another name: 2-hydroxypropyl(meth)acrylate), 2-hydroxy-1-methylethyl(meth)acrylate, hydroxypropyl(meth)acrylate, 2,3-dihydroxypropyl(meth)acrylate, and hydroxyethyl(meth)acrylate.

The carboxylic acid group-containing compound is not particularly limited, and examples thereof include a copolymer of any one or two or more monomers among olefinic monomers such as ethylene, propylene, and butylene, diene-based monomers such as butadiene, aromatic group-containing monomers such as styrene, and (meth)acrylic acid ester-based monomers, and a monomer having a carboxylic acid group [-COOH] such as (meth)acrylic acid; homopolymer of monomer having carboxylic acid group such as (meth)acrylic acid; and amino acid.

The sulfonic acid group-containing compound is not particularly limited, and examples thereof include a copolymer of styrene and acrylamide-2-methylpropane sulfonic acid (salt); ternary copolymer of styrene, n-butyl acrylate, and acrylamide-2-methylpropane sulfonic acid (salt); and ternary copolymer of styrene, 2-ethylhexyl acrylate, and acrylamide-2-methylpropane sulfonic acid (salt).

The ether group-containing compound is not particularly limited, and examples thereof include polyethylene glycol and derivatives thereof, fluororesins having an ether group, polyurethane resins having an ether group, and polyphenylene resins having an ether group.

The epoxy group-containing compound is not particularly limited, and examples thereof include epoxy resins, modified epoxy resins, (meth)acrylic (co)polymers having an epoxy group, polybutadiene resins having an epoxy group, polyurethane resins having an epoxy group, and adducts or condensates of these resins.

The amino group-containing compound is not particularly limited, and examples thereof include polyethyleneimine, polyvinylamine, polyamidopolyamine, polyamidine, polydimethylaminoethyl methacrylate, and polydimethylaminoethyl acrylate.

The amide group-containing compound is not particularly limited, and examples thereof include poly(N-isopropyl(meth)acrylamide) and poly(N-vinyl-2-pyrrolidone).

The phosphorylcholine group-containing compound is not particularly limited, and examples thereof include poly((meth)acryloyloxyethyl phosphorylcholine) which is a monomer of 2-methacryloyloxyethyl phosphorylcholine, and a copolymer of (meth)acryloyloxyethyl phosphorylcholine and a (meth)acrylic monomer.

The weight average molecular weight of the hydrophilic compound is not particularly limited, but is preferably 100 to 1,000,000.

The time for immersing the filter in the solution of the hydrophilic compound is not particularly limited as long as the filter can be coated with the hydrophilic compound, and is preferably 1 second to 60 minutes, and more preferably 10 seconds to 30 minutes although depending on, for example, the concentration of the hydrophilic compound in the solution of the hydrophilic compound to be used.

The immersion temperature and atmosphere are not particularly limited, and may be appropriately selected according to, for example, the type of the hydrophilic compound.

In addition, when an aqueous solution is used as the solution of the hydrophilic compound, even when the filter that has not been subjected to any treatment is immersed in the aqueous solution of the hydrophilic compound, the hydrophilic compound may not penetrate into the filter. Therefore, before immersing the filter in the solution of the hydrophilic compound, for example, it is preferable to immerse the filter in a solvent compatible with water such as isopropyl alcohol (impregnate the filter with a solvent compatible with water).

When the filter is immersed in a solvent compatible with water, it is preferable that the filter immersed in the solvent compatible with water is taken out from the solvent, and then the filter is immersed in a solution of a hydrophilic compound to replace the solvent compatible with water and the hydrophilic compound. In this case, the hydrophilic compound may be pushed into the filter by applying mechanical strength to the filter. Specifically, the filter may be easily impregnated with the hydrophilic compound by pressing and rubbing, or by pressure reduction or pressurization using a vacuum pressurization impregnation apparatus.

The solvent compatible with water is not particularly limited, but a solvent that easily penetrates into the filter and easily volatilizes is preferable, and specific examples thereof include alcohols such as methyl alcohol, ethyl alcohol, n-propyl alcohol, isopropyl alcohol, n-butyl alcohol, sec-butyl alcohol, tert-butyl alcohol, and isobutyl alcohol; esters such as methyl acetate, ethyl acetate, and butyl acetate; ketones such as acetone and methyl ethyl ketone; ethers such as tetrahydrofuran and dioxane; and aprotic polar solvents such as dimethyl sulfoxide and N,N-dimethylformamide. Among these, methyl alcohol, ethyl alcohol, or isopropyl alcohol is preferable from the viewpoint of, for example, easy penetration into the filter.

In addition, the solvent compatible with water may be a liquid obtained by mixing water with at least one selected from the alcohols, esters, ketones, ethers, and aprotic polar solvents.

These solvents compatible with water may be used singly or in combination of two or more types thereof.

The time for immersing the filter in a solvent compatible with water is not particularly limited, and is, for example, 1 minute to 24 hours.

The immersion temperature and atmosphere are not particularly limited.

Examples of the method for crosslinking the hydrophilic compound include irradiation crosslinking by an ionizing radiation such as an electron beam, thermal crosslinking, and chemical crosslinking using a crosslinking agent. Among these crosslinking methods, chemical crosslinking using a crosslinking agent is preferable because crosslinking can be performed even in an aqueous solution, and from the viewpoint of, for example, reliability of crosslinking.

The crosslinking agent to be used in the chemical crosslinking is not particularly limited, and may be appropriately selected according to the type of hydrophilic compound to be used, and examples thereof include aldehyde-based compounds such as formaldehyde, glutaraldehyde, and terephthalaldehyde; ketone compounds such as diacetyl and chloropentanedione; a compound having a reactive halogen such as bis(2-chloroethylurea)-2 hydroxy-4,6-dichloro-1,3,5-triazine; a compound having reactive olefin such as divinylsulfone; N-methylol compound; isocyanates; aziridine compounds; carbodiimide-based compound; epoxy compound; halogen carboxyaldehydes such as mucochloric acid; dioxane derivatives such as dihydroxydioxane; inorganic crosslinking agents such as chromium alum, zirconium sulfate, boric acid, borate, and phosphate; diazo compound such as 1,1-bis(diazoacetyl)-2-phenylethane; a compound containing disuccinimidyl ester; and difunctional maleimides.

These crosslinking agents may be used singly or in combination of two or more types thereof.

The crosslinking agent is preferably used in an amount that is greatly excessive with respect to the amount of the hydrophilic compound in the solution of the hydrophilic compound used.

From the viewpoint of, for example, easily obtaining a filter excellent in chemical resistance, the temperature at the time of crosslinking is preferably 10 to 95°C, and more preferably 20 to 60°C.

The crosslinking time at the time of crosslinking is preferably 1 to 60 minutes from the viewpoint of, for example, durability and productivity of the obtained filter.

The hydrophilized filter is preferably cleaned with, for example, water for the purpose of, for example, removing unreacted hydrophilic compounds. This step may be performed under heating as necessary.

The filter is preferably a filter that is transparent or translucent when wetted with the sample solution. Being transparent or translucent when wetted with a sample solution means that, for example, the average transmittance (hereinafter also referred to as "water-immersion average transmittance") of the filter immersed in water at 25°C for 1 minute at a wavelength of 400 to 700 nm is 20% or more. When the filter is transparent or translucent when wetted with the sample solution, for example, the position of the pipette tip, the state where, for example, the sample solution is sucked and discharged, and the state where the sample solution and the carrier are stirred are easily visually recognized from the outside of the container. Therefore, it becomes easier to cope with the automation apparatus.

Examples of the filter that is transparent or translucent when wetted with the sample solution include a filter made of an ultrafine fiber of PTFE and made of a nonwoven fabric subjected to a hydrophilic treatment. The filter is not only excellent in transparency but also excellent in liquid permeability, and thus the sample solution and the carrier can be easily diffused and mixed in the container, which is preferable.

The water immersion average transmittance is measured by the following method.

A test piece made of the filter is placed on an inner wall of a 10 mm square measurement cell made of quartz glass, and pure water is filled in the cell. The transmittance after 1 minute from the filling of pure water (transmittance of light perpendicularly incident on the wall surface of the test piece) is measured for each wavelength of 10 nm using an ultraviolet-visible spectrophotometer (UV-2600, manufactured by SHIMADZU CORPORATION), and the average transmittance (water immersion average transmittance) of the three test pieces at a wavelength of 400 to 700 nm is calculated.

### [Step 1]

Step 1 is a step of obtaining a container (ii) having a carrier to which the substance α or the substance β in the sample solution is adsorbed, in the first chamber. The container (i) becomes the container (ii) when the first chamber has a carrier to which the substance α or the substance β in the sample solution is adsorbed.

### [Carrier]

The carrier is a substance for adsorbing the substance α or the substance β in the sample solution.

When a purified liquid having an increased purity of the substance α is produced from a sample solution containing the substance α, the carrier has adsorbability for the substance α, and preferably has non-adsorbability for impurities. For example, when a purified liquid having an increased purity of a protein is produced from a sample solution containing the protein, the carrier has adsorbability for the protein.

Here, the term "adsorbability" means a property of adsorbing under a specific adsorption condition, and the term "non-adsorbability" means non-adsorbability under a specific condition.

Examples of the carrier having adsorbability for proteins include clay, agarose, dextran, hydroxyapatite, silica gel, polystyrene, phenol resin, acrylic resin, polyolefin (for example, polyethylene and polypropylene), polyvinyl chloride, and derivatives thereof. Examples of the derivative of agarose include Sepharose, examples of the derivative of dextran include Sephadex, and examples of the derivative of hydroxyapatite include carbonate hydroxyapatite. Among these, hydroxyapatite, Sepharose, and agarose are preferable, and hydroxyapatite is more preferable.

Examples of the carrier having adsorbability for nucleic acids include hydroxyapatite, silica, magnetic silica beads, cellulose, and agarose or beads immobilized with a complementary strand or a substance having structural affinity for nucleic acids.

Examples of the carrier having adsorbability for lipids include agarose or beads immobilized with Tim4 that binds to phosphatidylserine.

Examples of the carrier having adsorbability for saccharides include an ion exchange resin, activated carbon, agarose or beads immobilized with lectins or sugar chain-recognizing antibodies, boronic acid, and derivatives thereof.

In the "production method (X)-2", examples of the carrier having adsorbability for exosomes include agarose or beads immobilized with substances having affinity for molecules expressed on the exosome surface, and more specific examples thereof include agarose or beads immobilized with lipid-binding proteins such as Tim4 that binds to phosphatidylserine, lipid-recognizing antibodies, or lipid-recognizing aptamers; agarose or beads immobilized with antibodies or aptamers that recognize exosome-specific proteins such as CD9 and other proteins from the tetraspanin family; and agarose or beads immobilized with molecules that recognize lectins.

In the "production method (X)-2", examples of the carrier having adsorbability for viruses include agarose or beads (for example, agarose or beads immobilized with antibodies or aptamers recognizing viral spike proteins) immobilized with substances having affinity for molecules expressed on the surface of viruses.

When proteins are adsorbed to the carrier, a combination of substances having specific affinity may be used to adsorb proteins. That is, the carrier may be one immobilized with one of combinations of substances having specific affinity via a chemically modifying group. Examples of the combination of substances having specific affinity include an antibody and an antigen, an antibody and an antibody-binding protein, a glutathione-S-transferase (GST) tag and glutathione, a His tag and a divalent cation, and biotin and avidin.

When a purified liquid having an increased purity of the substance α is produced from a sample solution containing the substance β, the carrier has adsorbability for the substance β, preferably an encapsulated substance, and may have adsorbability or non-adsorbability for the substance α**.** For example, when a purified liquid having an increased purity of nucleic acid is produced from a sample solution containing a substance containing nucleic acids, the carrier is preferably a carrier having adsorbability for a substance containing nucleic acids, preferably an encapsulated substance, and the carrier may be a carrier having non-adsorbability for nucleic acids or a carrier having adsorbability for nucleic acids.

In addition, when a carrier having adsorbability for the substance α and the substance β is used as the carrier in the case of producing a purified liquid having an increased purity of the substance α from a sample solution containing the substance β, the adsorbability between the carrier and the substance β may be enhanced more than the adsorbability between the carrier and the substance α, or the carrier and the substance β may be adsorbed, and the carrier and the substance α may not be adsorbed by adjusting the conditions when the carrier and the substance β are brought into contact with each other.

Examples of carriers having adsorbability for substances containing nucleic acids, preferably encapsulated substances, and more preferably proteins, as well as adsorbability for nucleic acids, include hydroxyapatite.

When a purified liquid having an increased purity of nucleic acid is produced from a sample solution containing viruses, the carrier preferably has adsorbability for at least one selected from proteins, lipids, and sugars constituting the encapsulated substance, and more preferably has adsorbability for proteins. In this case, the carrier may be a carrier having non-adsorbability for nucleic acids or a carrier having adsorbability for nucleic acids.

When a purified liquid having an increased purity of the substance α, which is at least one selected from proteins, lipids, and sugars, is produced from a sample solution containing extracellular vesicles, the carrier preferably has adsorbability for at least one selected from proteins, lipids, and sugars constituting the encapsulated substance, and more preferably has adsorbability for proteins. In this case, the carrier may be a carrier having non-adsorbability for the substance α or a carrier having adsorbability for the substance α.

In the "production method (X)-2", when a purified liquid having an increased purity of viruses, extracellular vesicles, or DDS preparations is produced from a sample solution containing the viruses, the extracellular vesicles, or the DDS preparations, the carrier preferably has adsorbability for at least one selected from proteins, lipids, and sugars that constitute an encapsulated substance of the viruses, the extracellular vesicles, or the DDS preparations, more preferably has adsorbability for lipids, further preferably has adsorbability for phospholipids, and particularly preferably has adsorbability for phosphatidylserine.

Examples of the carrier having adsorbability for phosphatidylserine constituting the encapsulated substance of exosomes include agarose or beads immobilized with a substance having affinity for phosphatidylserine (for example, agarose or beads immobilized with lipid-binding proteins such as Tim4 that bind to phosphatidylserine, lipid-recognizing antibodies, or lipid-recognizing aptamers).

Examples of the carrier having adsorbability for proteins constituting the encapsulated substance of exosomes include agarose or beads immobilized with antibodies or aptamers that recognize exosome-specific proteins, such as CD9 and other proteins from the tetraspanin family.

Examples of the carrier having adsorbability for sugars constituting the encapsulated substance of exosomes include agarose or beads immobilized with molecules that recognize lectins.

When a purified liquid having an increased purity of a substance α which is at least one selected from nucleic acids, proteins, lipids, and sugars is produced from a sample solution containing liposome, it is preferable that the carrier has adsorbability for lipids constituting the encapsulated substance and has non-adsorbability for the substance α**.**

Examples of the carrier having non-adsorbability for nucleic acids and adsorbability for proteins include agarose or beads immobilized with protein-recognizing antibodies.

Examples of the carrier having non-adsorbability for nucleic acids and adsorbability for lipids include agarose or beads immobilized with lipid-binding proteins such as Tim4 or lipid-recognizing antibodies.

Examples of the carrier having non-adsorbability for nucleic acids and adsorbability for saccharides include agarose or beads immobilized with lectins or sugar chain-recognizing antibodies.

Examples of the carrier having non-adsorbability for proteins and adsorbability for lipids include agarose or beads immobilized with lipid-binding proteins such as Tim4 or lipid-recognizing antibodies.

Examples of the carrier having non-adsorbability for proteins and adsorbability for saccharides include agarose or beads immobilized with lectins or sugar chain-recognizing antibodies.

Examples of the carrier having non-adsorbability for lipids and adsorbability for nucleic acids include silica and hydroxyapatite.

Examples of the carrier having non-adsorbability for lipids and adsorbability for proteins include agarose or beads immobilized with antibodies, and hydroxyapatite.

Examples of the carrier having non-adsorbability for lipids and adsorbability for saccharides include agarose or beads immobilized with lectins or sugar chain-recognizing antibodies.

Examples of the carrier having non-adsorbability for saccharides and adsorbability for nucleic acids include silica and hydroxyapatite.

Examples of the carrier having non-adsorbability for saccharides and adsorbability for proteins include agarose or beads immobilized with antibodies, and hydroxyapatite.

Examples of the carrier having non-adsorbability for saccharides and adsorbability for lipids include agarose or beads immobilized with lipid-binding proteins such as Tim4 or lipid-recognizing antibodies.

When viruses or extracellular vesicles are adsorbed to the carrier, the antigen proteins present on the surface of viruses or the extracellular vesicles may be used to adsorb the viruses or extracellular vesicles to the carrier. Examples of the antigenic protein present on the exosome surface include tetraspanins such as CD9, CD63, and CD81; antigen presentation-related proteins such as MHCI and MHCII; adhesion molecules such as integrin, ICAM-1, and EpCAM; cytokines/cytokine receptors such as EGFRvIII and TGF-β; and enzymes.

The carrier may be used singly or in combination of two or more types thereof.

The shape of the carrier is not particularly limited, and examples thereof include a spherical shape, a particulate shape, a fibrous shape, a rod shape, and a plate shape, but a spherical shape and a particulate shape are preferable from the viewpoint of ease of solid-liquid separation and cleaning.

The size of the carrier is not particularly limited as long as the carrier is larger than the pores of the filter, and varies depending on the type of the substance α**,** but when the carrier is spherical or particulate, the average particle diameter thereof is preferably 0.5 µm to 2 mm, more preferably 1 µm to 1.5 mm, and still more preferably 1 µm to 1 mm. When the average particle diameter of the carrier is within the above range, the carrier and the sample solution are easily separated. In addition, the method for measuring the average particle diameter can be performed using a light scattering method.

The amount of the carrier used in the production method (X) is not particularly limited, and may be appropriately selected according to, for example, the type of the carrier, the type of the sample solution, or the amounts of the substance α and the substance β in the sample solution.

### [Method for Obtaining Container (ii)]

In the step 1, the method for obtaining the container (ii) is not particularly limited, but it is preferable to obtain the container (ii) by any one of the following steps a to d.

Step a: a step of adsorbing the substance α or the substance β to the carrier by putting the sample solution into the second chamber and diffusing the substance α and the substance β in the sample solution, after a carrier capable of adsorbing the substance α or the substance β is put into the first chamber (here, the filter allows the substance α and the substance β to pass therethrough).

Step b: a step of adsorbing the substance α or the substance β to the carrier by putting the sample solution into the first chamber and diffusing the substance α and the substance β in the sample solution, after a carrier capable of adsorbing the substance α or the substance β is put into the first chamber.

Step c: a step of adsorbing a substance containing the substance α or the substance β to the carrier by providing the first chamber having a carrier capable of adsorbing the substance α or the substance β and the second chamber, and diffusing the substance α and the substance β in the sample solution, in a container (iii), after the sample solution is put into the container (iii) having an opening in the upper portion (here, the filter allows the substance α and the substance β to pass therethrough).

Step d: a step of putting a carrier to which the substance α or the substance β is adsorbed into the first chamber after obtaining a carrier to which a substance containing the substance α or the substance β is adsorbed, by mixing the sample solution with a carrier capable of adsorbing the substance α or the substance β, and diffusing the substance α and the substance β in the sample solution.

### [Step a]

In the step a, the substance α or the substance β in the sample solution put into the second chamber diffuses in the sample solution, passes through the filter A, reaches the first chamber, and is adsorbed to the carrier in the first chamber. Since this series of flows is repeated not once but a plurality of times, the substance α or the substance β is more easily adsorbed to the carrier as compared with a conventional method by column chromatography, filtration, or centrifugation in which the chance of adsorption to the carrier is only once. Therefore, a purified liquid having a higher purity of the substance α can be easily produced.

On the other hand, among impurities in the sample solution put into the second chamber, impurities larger than the pore diameter of the filter A cannot pass through the filter A and remain in the second chamber, and impurities smaller than the pore diameter of the filter A pass through the filter A and reach the first chamber. However, by using a carrier that specifically adsorbs the substance α or the substance β as the carrier, impurities are not adsorbed to the carrier and remain in the sample solution. Therefore, when the removal step of removing the liquid present in the container (ii) after the step a is performed, the impurities can be removed by being separated from the carrier to which the substance α or the substance β is adsorbed in the removal step. Therefore, after this, when the releasing solution is brought into contact with the carrier to release the substance α from the carrier, a purified liquid having an increased purity of the substance α can be obtained.

### [Step b]

In the step b, the substance α or the substance β in the sample solution put into the first chamber diffuses in the sample solution, and is adsorbed to the carrier in the first chamber. Since this series of flows is repeated not once but a plurality of times, the substance α or the substance β is more easily adsorbed to the carrier as compared with a conventional method by column chromatography, filtration, or centrifugation in which the chance of adsorption to the carrier is only once. Therefore, a purified liquid having a higher purity of the substance α can be easily produced.

On the other hand, among the impurities in the sample solution put into the first chamber, impurities larger than the pore diameter of the filter cannot pass through the filter and remain in the first chamber. However, by using a carrier that specifically adsorbs the substance α or the substance β as the carrier, the impurities are not adsorbed to the carrier and remain in the sample solution, and impurities smaller than the pore diameter of the filter pass through the filter and reach the second chamber. Therefore, when the removal step of removing the liquid present in the container (ii) after the step b is performed, the impurities can be removed by being separated from the carrier to which the substance α or the substance β is adsorbed in the removal step. Therefore, after this, when the releasing solution is brought into contact with the carrier to release the substance α from the carrier, a purified liquid having an increased purity of the substance α can be obtained.

### [Step c]

In the step c, after the sample solution is put into the container (iii) having an opening in an upper portion, the first chamber having a carrier capable of adsorbing the substance α or the substance β and having an opening in an upper portion and a second chamber partitioned from the first chamber by a filter and having an opening in an upper portion are provided in the container (iii).

A method for providing the first chamber and the second chamber in the container (iii) is not particularly limited, and examples thereof include a method (I) in which the container (iii) is simply partitioned by one or two or more of the filters, and one chamber in the partitioned container (iii) is defined as a first chamber and the other part in the container (iii) is defined as a second chamber, and a method (II) in which a member of bag, cylindrical, or other shape having an opening in an upper portion and at least a part of the wall formed by the filters is put into the container (iii), a space in the member of bag, cylindrical, or other shape is defined as a first chamber, and a space other than the member of bag, cylindrical, or other shape in the container (iii) is defined as a second chamber. Among these, the method (II) is preferable from the viewpoint that, for example, the desired first chamber and second chamber can be easily provided.

In addition, in the case of the method (I), usually, the first chamber and the second chamber are provided, and then the carrier is put into the first chamber. In the case of the method (II), a member of bag, cylindrical, or other shape containing the carrier may be put into the container (iii), or the carrier may be put into the member after a member of bag, cylindrical, or other shape containing the carrier is put into the container (iii).

In the step c, the substance α or the substance β in the sample solution put into the container (iii) diffuses in the sample solution, and is adsorbed to the carrier in the first chamber. Since this series of flows is repeated not once but a plurality of times, the substance α or the substance β is more easily adsorbed to the carrier as compared with a conventional method by filtration or centrifugation in which the chance of adsorption to the carrier is only once. Therefore, a purified liquid having a higher purity of the substance α can be easily produced.

On the other hand, for example, in the method (II), among impurities in the sample solution put into the container (iii), impurities larger than the pore diameter of the filter A cannot pass through the filter A and remain in the second chamber, and impurities smaller than the pore diameter of the filter A pass through the filter A and reach the first chamber. However, by using a carrier that specifically adsorbs the substance α or the substance β as the carrier, impurities are not adsorbed to the carrier and remain in the sample solution. Therefore, when the removal step of removing the liquid present in the container (iii) after the step c is performed, the impurities can be removed by being separated from the carrier to which the substance α or the substance β is adsorbed in the removal step. Therefore, after this, when the releasing solution is brought into contact with the carrier to release the substance α from the carrier, a purified liquid having an increased purity of the substance α can be obtained.

### [Step d]

In step d, the sample solution and the carrier are mixed first, and the substance α or the substance β can be sufficiently adsorbed to the carrier. Therefore, the substance α or the substance β is more easily adsorbed to the carrier as compared with the conventional method by filtration or centrifugation in which the chance of adsorption to the carrier is only once. Therefore, a purified liquid having a higher purity of the substance α can be easily produced.

In addition, not only the carrier to which the substance α or the substance β is adsorbed, but also the residual liquid remaining after the carrier to which the substance α or the substance β is adsorbed is obtained by mixing the sample solution with the carrier capable of adsorbing the substance α or the substance β and diffusing the substance α and the substance β in the sample solution may be put into the first chamber. Further, the carrier to which the substance α or the substance β is adsorbed may be separated from the residual liquid and then cleaned, and then put into the first chamber, or the carrier to which the substance α or the substance β is adsorbed may be separated from the residual liquid and then cleaned as necessary, and then put into the first chamber together with a dispersion medium for dispersing the carrier.

### [Method for Diffusing Substance α and Substance β]

The method for diffusing the substance α and the substance β in the sample solution in steps a to d is not particularly limited, and either diffusion by thermal motion of molecules (molecular diffusion) or dispersion by convection (turbulent diffusion) may be used, but a method for promoting convection such as stirring, heating, or ultrasonic waves is preferable, and stirring is more preferable. Examples of the method for stirring includes a method for rotating a stirrer in a sample solution is used; a method for sending, for example, air into sample solution; and a method for moving the liquid surface of the sample solution up and down by an operation using, for example, a vortex mixer, or a pipetting operation.

Since the substance α and the substance β can be efficiently adsorbed to the carrier and an automation apparatus can be used, the method for diffusing the substance α and the substance β in the sample solution in steps a to d is preferably a method for moving the liquid surface of the sample solution up and down, and is more preferably performed by a pipetting operation.

In the case of the steps a to c, the pipetting operation may be performed in any one of the first chamber and the second chamber, and which one of the first chamber and the second chamber is performed may be appropriately determined according to, for example, a type and a size of a conceivable impurities. Since it is possible to avoid suction and discharge of the carrier, it is preferable to perform the operation in the second chamber.

The condition of the pipetting operation is not particularly limited, but the volume to be sucked and discharged is preferably 50% or more, more preferably 60% or more, and still more preferably 70% or more of the sample solution.

The pipetting operation is preferably performed at a frequency of 1 time/min or more, more preferably 1.5 times/min or more, and still more preferably 2 times/min or more, and is preferably performed for 10 minutes to 24 hours, more preferably 15 minutes to 12 hours, and still more preferably 30 minutes to 1 hour.

When the pipetting operation is performed under the above conditions, the substance α and the substance β can be efficiently adsorbed to the carrier.

The instrument or apparatus that performs the pipetting operation is not particularly limited, and for example, a pipette such as a micropipette, a macropipette, a measuring pipette, or a Komagome pipette can be used, but from the viewpoint of safety and contamination prevention, a micropipette or a macropipette using a disposable tip is preferable.

The instrument or apparatus that performs the pipetting operation may be manual or electric, but an electric pipette is preferable because the pipetting operation can be accurately performed.

### [Step 2-1]

Step 2-1 is a step of obtaining a purified liquid having an increased purity of the substance α by bringing a liquid capable of releasing the substance α (the releasing solution which will be described later) from the carrier into contact with the carrier and releasing the substance α from the carrier, in the container (ii).

The method for performing the step 2-1 is not particularly limited, but for example, the releasing solution is added dropwise onto the carrier. Since the operation is simple and copes with an automation apparatus, the method is preferably performed by a pipetting operation.

### [Step 2-2]

Step 2-2 is a step of obtaining a purified liquid having an increased purity of the substance α by bringing a releasing solution which will be described later into contact with the carrier to release the substance α from the carrier.

The method for performing the step 2-2 is not particularly limited, but for example, the releasing solution is added dropwise onto the carrier. Since the operation is simple and copes with an automation apparatus, the method is preferably performed by a pipetting operation.

The step 2-2 may be performed in the container (ii) or in a container other than the container (ii) (hereinafter referred to as a vessel A).

When it is not preferable to release the substance α from the carrier in the container (ii), such as when impurities not dispersed or dissolved in the sample solution are present in the container (ii) (for example, adherent cells and others are adhered to the inner wall of the container), or when the container (ii) is excessively large with respect to the amount of releasing solution, the step 2-2 is preferably performed in the vessel A. When impurities which are not dispersed or dissolved in the sample solution are present in the container (ii) and the substance α is released from the carrier in the container (ii), there is a possibility that the impurities are dispersed or dissolved in the purified liquid and mixed into the purified liquid. When the step 2-2 is performed in the vessel A, even when impurities not dispersed in the sample solution are present in the container (ii), the impurities can be prevented from being mixed into the purified liquid. In addition, even when the amount of the releasing solution is small, the purified liquid can be efficiently recovered.

When the step 2-2 is performed in the vessel A, usually, after the step 1 and before the step 2-2, the carrier to which the substance α or the substance β is adsorbed, the filter, and at least a part of the member constituting the first chamber is transferred from the container (ii) into the vessel A while the carrier to which the substance α or the substance β is adsorbed is contained in the first chamber.

Since pipetting operations inside the vessel A, particularly processes such as facilitating the insertion of the pipette tip, can be easily performed, it is also preferable to transfer the spacer into the vessel A when a spacer is inserted into the first chamber.

When the first chamber and the second chamber are provided in the container (iii) by, for example, the method (II) in which, when obtaining the container (ii) by the step c, in the step c, a member of bag, cylindrical, or other shape having an opening in the upper portion and at least a part of the wall composed of the filter is put into the container (iii), the space within the member of bag, cylindrical, or other shape is defined as a first chamber, and the space in the container (iii) other than the member of bag, cylindrical, or other shape is defined as a second chamber, the transfer can be performed by transferring the member of bag, cylindrical, or other shape to the vessel A while the carrier to which the substance α or the substance β is adsorbed is still contained within the member.

In the "production method (X)-2", when the step 2-2 is performed in the vessel A, the container (ii) may be a container after being used for culturing, for example, cells.

The vessel A is not particularly limited as long as the vessel is a container different from the container (ii), and can be appropriately selected according to the amount of releasing solution.

The shape of the vessel A is not particularly limited, but a bottomed tubular container such as a test tube or a microtube is preferable because it is easy to perform a pipetting operation. In the case A, the carrier to which the substance α or the substance β is adsorbed, the filter, and at least a part of the member constituting the first chamber is transferred into the vessel A in a state where the carrier to which the substance α or the substance β is adsorbed is contained in the first chamber, and the vessel A preferably has an opening in the upper portion in order to input releasing solution, and the opening in the upper portion is preferably closable in order to prevent contamination.

The size of the vessel A is not particularly limited, and may be appropriately selected according to the type and amount of the releasing solution. The volume of the container is preferably 0.2 mL to 50 mL, more preferably 0.5 mL to 30 mL, and still more preferably 1 mL to 20 mL because the pipetting operation is easily performed.

The material constituting the vessel A may be appropriately selected according to the type of the sample solution, and is not particularly limited as long as the material has non-adsorbability for the substance α and does not have reactivity with the releasing solution, but examples thereof include synthetic resins such as polystyrene, polycarbonate, polyethylene, polypropylene, polyethylene terephthalate, polyetherimide, polyimide, ABS resin, polyvinyl chloride, polyvinylidene chloride, and fluororesin; glass; and metal such as stainless steel. These may be used singly, or in combination of two or more types thereof.

The vessel A is preferably a transparent or translucent container. When the vessel A is transparent or translucent, for example, the position of the pipette tip and the state where, for example, the releasing solution is sucked and discharged are easily visually recognized from the outside of the vessel A. Therefore, it becomes easier to cope with the automation apparatus.

The instrument or apparatus for performing the pipetting operation is the same as the instrument or apparatus described in the section of "Method for Diffusing Substance α and Substance β".

### [Liquid Capable of Releasing Substance α from Carrier]

Hereinafter, the liquid capable of releasing the substance α from the carrier is also referred to as "releasing solution".

The composition and properties, for example, of the releasing solution are not limited as long as the substance α can be released from the carrier.

Releasing the substance α from the carrier means that, for example, in a case where a substance in which the substance α is contained inside the encapsulated substance is used as the substance β, the encapsulated substance adsorbed to the carrier may or may not be released from the carrier, and in a case where the encapsulated substance is also released from the carrier, a separation operation between the substance α and the encapsulated substance may be required, and thus it is preferable not to release the substance α from the carrier because such a separation operation is omitted. Specifically, it is preferable to release the substance α by bringing a liquid (for example, nucleic acid extract) capable of releasing the substance α (for example, nucleic acid) into the liquid from the substance β (for example, encapsulated substance containing nucleic acid) adsorbed to the carrier into contact with the carrier.

When the substance α is adsorbed to the carrier, the substance α can be released from the carrier by changing the adsorbability of the carrier for the substance α**.**

For example, when the protein is adsorbed to hydroxyapatite, examples of the liquid capable of releasing the protein from hydroxyapatite include a metal chelating agent such as EDTA of 100 mM or more, a surfactant such as SDS or TritonX-100, and an aqueous solution of, for example, phosphoric acid of 100 mM or more. These may be used singly, or in combination of two or more types thereof.

When the substance β is a substance in which the substance α is contained inside the encapsulated substance, the substance α can be released from the substance β adsorbed to the carrier into the liquid by modifying or breaking the encapsulated substance. For example, when viruses or exosomes in which nucleic acids are contained inside proteins are adsorbed to a carrier, the nucleic acids can be released from the viruses or exosomes adsorbed to the carrier by using the releasing solution containing a protein denaturant.

Examples of the protein denaturant include surfactants such as SDS and TritonX-100; guanidine thiocyanate, guanidine hydrochloride, urea, chaotropic agent such as NaI; proteolytic enzymes such as Proteinase K; and organic solvent such as phenol. These may be used singly, or in combination of two or more types thereof. The protein denaturant may be, for example, a nuclease inhibitor such as EDTA; a reducing agent such as 2-mercaptoethanol or DTT may be used in combination. As the protein denaturant, for example, TRIzol Reagent, QIAzol Lysis Reagent, or ISOGEN commercially available as a nucleic acid extraction reagent may be used.

In the "production method (X)-2", for example, when viruses, extracellular vesicles, or DDS preparations are adsorbed to agarose or beads (carrier) immobilized with substances having affinity for molecules expressed on the surface of viruses, extracellular vesicles, or DDS preparations, the viruses, the extracellular vesicles, or the DDS preparations can be released from the carrier by changing a binding state between the molecule expressed on the surface of the viruses, the extracellular vesicles, or the DDS preparations and the substances having affinity.

For example, when the exosomes are adsorbed to agarose or beads (carrier) immobilized with lipid-binding proteins such as Tim4 that binds to phosphatidylserine expressed on the exosome surface, lipid-recognizing antibodies, or lipid-recognizing aptamers, the exosomes can be released from the carrier by changing the binding state of lipid-binding proteins such as Tim4, lipid-recognizing antibodies, or lipid-recognizing aptamers. Examples of the liquid capable of releasing the exosomes from the carrier include metal chelating agents such as EDTA in an amount of 0.5 mM or more.

When the substance α is released from the carrier as described above, a purified liquid containing viruses, extracellular vesicles, or DDS preparations in an intact state and having an increased purity of the viruses, the extracellular vesicles, or the DDS preparations can be produced from a sample solution containing the viruses, the extracellular vesicles, or the DDS preparations.

The amount of the releasing solution used is not particularly limited as long as the substance α can be sufficiently released from the carrier. It may be appropriately determined according to, for example, the amount of the substance α or the substance β adsorbed to the carrier.

As the releasing solution, a plurality of different releasing solutions may be sequentially brought into contact with the carrier. Accordingly, it is possible to further increase the purity of the substance α contained in the purified liquid.

In addition, the releasing solution after being brought into contact with the carrier may be brought into contact with the carrier again. Accordingly, it is possible to further increase the purity of the substance α contained in the purified liquid.

### [Removal Step]

The production method (X) preferably includes a step of removing a liquid present in the container (ii) after any one of the steps a to d, after any one of the steps a to d and before bringing the liquid capable of releasing the substance α from the carrier into contact with the carrier. Hereinafter, this step is also referred to as "removal step".

Since the liquid present in the container (ii) after any one of the steps a to d contains a large amount of impurities not adsorbed to the carrier, the impurities in the container (ii) can be reduced by removing the liquid.

The method for performing the removal step is not particularly limited, and examples thereof include an aspiration operation and a pipetting operation. Since an automation apparatus can be used and the liquid can be gently removed, the removal step is preferably performed by a pipetting operation.

The removal step may be performed in either the first chamber or the second chamber, but is preferably performed in the second chamber because the carrier can be avoided from being removed.

The instrument or apparatus for performing the pipetting operation is the same as the instrument or apparatus described in the section of "Method for Releasing Substance α from Carrier".

As the production method (X), since an automation apparatus can be used, preferably, any of the method for diffusing the substance α and the substance β in the sample solution, the step of removing the liquid present in the container (ii) after any one of the steps a to d, and the method for obtaining a purified liquid having an increased purity of the substance α by bringing a liquid capable of releasing the substance α from the carrier into contact with the carrier and releasing the substance α from the carrier is performed by a pipetting operation.

### [Cleaning Step]

The production method (X) may include a step of putting a cleaning fluid into the container (ii) and cleaning the container (ii) after the removal step and before bringing a liquid capable of releasing the substance α from the carrier into contact with the carrier. Hereinafter, this step is also referred to as "cleaning step".

Most of the impurities not adsorbed to the carrier can be removed from the inside of the container (ii) by the removal step, but a part of the impurities are likely to adhere to and remain on, for example, a wall, a filter, and a carrier in the container (ii). In particular, impurities larger than the pore diameter of the filter adhere to the outside of the filter, and the pores are likely to be clogged. Since impurities can be more efficiently removed by the cleaning step, when the amount of impurities is large, the production method (X) preferably includes the cleaning step.

The method for performing the cleaning step is not particularly limited, but the operation is simple and copes with an automation apparatus, the method is preferably performed by a pipetting operation.

The instrument or apparatus for performing the pipetting operation is the same as the "Method for Diffusing Substance α and Substance β".

The cleaning step may be performed in either the first chamber or the second chamber.

In the case of the container (ii) obtained by the method (II) in the step a or the step c, impurities larger than the pore diameter of the filter among impurities in the sample solution cannot pass through the filter and remain in the second chamber, and are likely to adhere to the surface of the filter on the second chamber side. Therefore, when a large amount of impurities larger than the pore diameter of the filter are contained in the sample solution, the filter can be backwashed, clogging of the pores can be easily eliminated, and thus the cleaning step is preferably performed in the first chamber. When no impurities larger than the pore diameter of the filter is contained in the sample solution or when a small amount of impurities is contained therein, the cleaning step and the step of removing the liquid present in the container (ii) can be performed in series without sucking and discharging the carrier, and thus the cleaning step is preferably performed in the second chamber.

In the case of the container (ii) obtained in the step b or the step d, on the contrary, impurities remain in the first chamber and are likely to adhere to the surface of the filter on the first chamber side. Therefore, the cleaning step is preferably performed in the second chamber.

The cleaning fluid is not particularly limited as long as it is difficult to release the substance α from the carrier, and examples thereof include water and a buffer solution. As the buffer solution, for example, a buffer solution usually used in a biochemical test can be used, and examples thereof include a Tris buffer solution and a phosphate buffer solution. The cleaning fluid may contain, as appropriate, salts such as sodium chloride; surfactants such as SDS; metal chelating agents such as EDTA; and preservatives such as sodium azide, depending on the type and amount of impurities. For example, in the case of producing a purified liquid having an increased purity of nucleic acid from a sample solution containing viruses, preferably using hydroxyapatite as a carrier, free nucleic acids derived from substances other than viruses can be removed by performing the cleaning step using a cleaning fluid containing EDTA.

The amount of the cleaning fluid is not particularly limited. The volume of the cleaning fluid may be appropriately determined according to the type and amount of impurities, and is preferably 20 times or more, more preferably 100 times or more, and still more preferably 200 times or more the volume of the carrier.

The cleaning fluid may sequentially contain a plurality of different cleaning fluids in the container (ii). Thus, impurities can be efficiently removed, and the purity of the substance α contained in the purified liquid can be further increased.

When the production method (X) includes the cleaning step, after the cleaning step, the cleaning fluid may be stirred, for example, by sucking and discharging the cleaning fluid put into the container (ii) by a pipetting operation, or by tapping the container (ii). Thus, impurities can be efficiently removed, and the purity of the substance α contained in the purified liquid can be further increased.

When the production method (X) includes the cleaning step, the production method preferably includes a step of removing a liquid present in the container (ii) after the cleaning step and before bringing a liquid capable of releasing the substance α into contact with the carrier. Thus, impurities can be efficiently removed, and the purity of the substance α contained in the purified liquid can be further increased.

### [Recovery Step]

The production method (X) may include a step of recovering the purified liquid obtained in the step 2-1 or the step 2-2 after the step 2-1 or the step 2-2. Hereinafter, this step is also referred to as "recovery step". When the recovery step is performed, the purified liquid can be transferred to a vessel other than the container (ii) used in the production method (X).

The method for performing the recovery step is not particularly limited, but the recovery step is preferably performed by a pipetting operation because an automation apparatus can be used and the purified liquid can be gently recovered.

The recovery step may be performed in either the first chamber or the second chamber, but is preferably performed in the second chamber because the carrier can be avoided from being recovered.

The instrument or apparatus for performing the pipetting operation is the same as the instrument or apparatus described in the section of "Method for Releasing Substance α from Carrier".

In the "production method (X)-2", when the step 2-2 is performed in the vessel A, the purified liquid is transferred to a vessel (that is, the vessel A) other than the container (ii) used in the production method (X) without performing the recovery step.

### <<Purification Kit>>

According to another aspect of the present invention, a purification kit for purifying a substance α from a sample solution containing a separation target substance (substance α) or a substance containing the separation target substance (substance β) is a purification kit including: a container, a carrier capable of adsorbing the substance α or the substance β, and a filter that does not allow the carrier to pass through, in which the filter is used to partition the container into a first chamber and a second chamber each having an opening in an upper portion, and the first chamber has the carrier. Hereinafter, this purification kit is also referred to as "purification kit (Y)".

When the purification kit (Y) is used, the method for using the purification kit (Y) is not limited as long as the filter partitions the container into a first chamber and a second chamber each having an opening in the upper portion, and the first chamber has the carrier. For example, it is possible to put the filter into a container having an opening in an upper portion, partition the inside of the container into a first chamber and a second chamber, and then put the carrier into the first chamber. In addition, a member of bag, cylindrical, or other shape having an opening in the upper portion and at least a part of the wall composed of the filter may be put into the container, the inside of the member of bag, cylindrical, or other shape may be defined as the first chamber, and a space other than the member of bag, cylindrical, or other shape in the container may be defined as the second chamber. In this case, a member of bag, cylindrical, or other shape containing the carrier may be put into the container, or the carrier may be put into the member after a member of bag, cylindrical, or other shape containing the carrier is put into the container.

The purification kit (Y) may contain the releasing solution in addition to a container, a carrier capable of adsorbing the substance α or the substance β, and a filter that does not allow the carrier to pass therethrough.

The purification kit (Y) may contain the cleaning fluid in addition to a container, a carrier capable of adsorbing the substance α or the substance β, and a filter that does not allow the carrier to pass therethrough.

The purification kit (Y) may contain, for example, other reagents or experimental instruments, for example, for sampling or storage.

### [Other Aspects]

Another aspect of the present invention is a method for adsorbing a substance α or a substance β to a carrier in a sample solution containing a separation target substance (substance α) or a substance containing the substance α (substance β), the method including any one of the following steps a to c.

Step a: a step of adsorbing the substance α or the substance β to the carrier by putting the sample solution into the second chamber and diffusing the substance α and the substance β in the sample solution, after a carrier capable of adsorbing the substance α or the substance β is put into the first chamber (here, the filter allows the substance α and the substance β to pass therethrough).

Step b: a step of adsorbing the substance α or the substance β to the carrier by putting the sample solution into the first chamber and diffusing the substance α and the substance β in the sample solution, after a carrier capable of adsorbing the substance α or the substance β is put into the first chamber.

Step c: a step of adsorbing a substance containing the substance α or the substance β to the carrier by providing the first chamber having a carrier capable of adsorbing the substance α or the substance β and the second chamber, and diffusing the substance α and the substance β in the sample solution, in a container (iii), after the sample solution is put into the container (iii) having an opening in the upper portion (here, the filter allows the substance α and the substance β to pass therethrough).

Another aspect of the present invention is a method for separating a substance α or a substance β from a sample solution containing a separation target substance (substance α) or a substance containing the substance α (substance β), the method including any one of the following steps a to d and a step of removing a liquid present in a container (ii) after any one of the steps a to d.

Step a: a step of adsorbing the substance α or the substance β to the carrier by putting the sample solution into the second chamber and diffusing the substance α and the substance β in the sample solution, after a carrier capable of adsorbing the substance α or the substance β is put into the first chamber (here, the filter allows the substance α and the substance β to pass therethrough).

Step b: a step of adsorbing the substance α or the substance β to the carrier by putting the sample solution into the first chamber and diffusing the substance α and the substance β in the sample solution, after a carrier capable of adsorbing the substance α or the substance β is put into the first chamber.

Step c: a step of adsorbing a substance containing the substance α or the substance β to the carrier by providing the first chamber having a carrier capable of adsorbing the substance α or the substance β and the second chamber, and diffusing the substance α and the substance β in the sample solution, in a container (iii), after the sample solution is put into the container (iii) having an opening in the upper portion (here, the filter allows the substance α and the substance β to pass therethrough).

Step d: a step of putting a carrier to which the substance α or the substance β is adsorbed into the first chamber after obtaining a carrier to which a substance containing the substance α or the substance β is adsorbed, by mixing the sample solution with a carrier capable of adsorbing the substance α or the substance β, and diffusing the substance α and the substance β in the sample solution.

### Examples

Next, the present invention will be described in more detail with reference to examples, but the present invention is not limited thereto.

### [Example 1]

A nonwoven fabric (weight: 16 g/m², 300 mm × 20 mm, average pore diameter: 1 µm) made of ultrafine PTFE fibers was formed into a bag shape (bag shape in which two nonwoven fabrics are stacked, three sides are sealed using double-sided tape (manufactured by Nichiban Co., Ltd., NICETACK strong type, #NW-K10), and the remaining one side is an open portion) having an inner diameter of 6 mm, a height of 45 mm, and a cylinder depth of 40 mm to prepare a PTFE filter bag. This PTFE filter bag was immersed in a 99.7% isopropyl alcohol (IPA) solution (manufactured by FUJIFILM Wako Pure Chemical Corporation) for 30 minutes at room temperature (25°C).

Next, the PTFE filter bag taken out from the IPA solution was immersed in 500 mL of a polyvinyl alcohol (PVA) (manufactured by FUJIFILM Wako Pure Chemical Corporation, 160-11485, polymerization degree: 1500, saponification degree: 98%) aqueous solution adjusted to a concentration of 0.1 mass% at room temperature for 30 minutes.

Thereafter, the PTFE filter bag taken out from the PVA aqueous solution was immersed in a mixed liquid obtained by mixing 500 mL of a 5 mass% glutaraldehyde solution (a solution prepared by diluting a 25% glutaraldehyde solution manufactured by FUJIFILM Wako Pure Chemical Corporation with pure water to adjust a concentration to 5 mass%) and 5 mL of a 36% aqueous hydrochloric acid solution (manufactured by FUJIFILM Wako Pure Chemical Corporation) at room temperature for 30 minutes.

Next, the PTFE filter bag taken out from the mixed liquid was put into pure water to dissolve unreacted IPA, PVA, and glutaraldehyde.

The PTFE filter bag taken out from the liquid was naturally dried to obtain a hydrophilized (PVA-treated) PTFE filter bag.

Dry hydrophilized (PVA-treated) PTFE filter bags were opaque and invisible to the contents, but were highly transparent in water.

A spiral-shaped spacer (diameter: 5 mm, length: 9.5 cm) made of stainless steel (manufactured by DAIDOHANT.CO.,LTD., yarn count: 30) was inserted into a hydrophilized (PVA-treated) PTFE filter bag in order to maintain the shape. A hydrophilized (PVA-treated) PTFE filter bag with a spacer was put into a 14 mL tube (manufactured by FALCON, model number: 352059), and the upper portion of the hydrophilized (PVA-treated) PTFE filter bag was fixed to the upper portion of the tube with a clip. At this time, the open portion of the hydrophilized (PVA-treated) PTFE filter bag was fixed to face the opening direction of the tube (to have an opening in the upper portion). That is, a container (tube) having a first chamber having an opening in the upper portion (a hydrophilized (PVA-treated) PTFE filter bag with a spacer having an opening in the upper portion), and a second chamber partitioned from the first chamber by a hydrophilic (PVA-treated) PTFE filter bag and having an opening in the upper portion (a part in a tube having an opening in the upper portion and outside the hydrophilized (PVA-treated) PTFE filter bag) was obtained.

Thereafter, into the hydrophilized (PVA-treated) PTFE filter bag, 50 µL of HT carrier particles (Bio-Gel HT Hydroxyapatite manufactured by Bio-Rad Laboratories, Inc., model number: 130-0150) was added with a micropipette.

A Cy3 fluorescent dye (manufactured by Amersham plc, model number: Q13108) was added to a bovine serum albumin solution (Sigma-Aldrich Co. LLC., 100 µg/10 µL, solvent: H₂O), and the mixture was reacted at room temperature in the dark for 1 hour. Thereafter, TBS was added to stop the reaction, and the mixture was allowed to stand at room temperature for 1 hour in the dark. Next, gel filtration (Thermo Fisher Scientific Inc., model number: 89883) was performed to remove the excess Cy3 fluorescent dye. Bovine serum albumin solution (25 µg/mL) labeled with a Cy3 fluorescent dye was prepared by dilution with PBS (manufactured by Takara Bio Inc., model number: T900).

4 mL of bovine serum albumin solution labeled with a Cy3 fluorescent dye was added into the tube, that is, to the outside of the hydrophilized (PVA-treated) PTFE filter bag, with a micropipette. This solution was defined as Input.

A pipette tip of an automatic pipettor (manufactured by Gilson, Inc., model number: P5000M, for 5000 µL) was put into the tube, that is, to the outside of the hydrophilized (PVA-treated) PTFE filter bag, perpendicularly to the liquid surface, and the automatic pipettor was disposed to allow suction and discharge of the solution. Note that the arrangement of the container (tube) and the automatic pipettor is a representation of an automation apparatus including only pipetting means for sucking and discharging a solution and means for moving the pipetting means up and down and back and forth.

Bovine serum albumin labeled with a Cy3 fluorescent dye was adsorbed to HT carrier particles by sucking and discharging 2800 µL of the solution at a speed of Speed 1 for 1 hour in a custom mode of the automatic pipettor.

Since the hydrophilized (PVA-treated) PTFE filter bag had high transparency in water, the position of the pipette tip and the state of sucking and discharging the solution could be visually recognized from the outside of the container.

Subsequent steps of adding and recovering the liquid were performed with a micropipette.

After the solution was sucked and discharged for 1 hour, the liquid present in the tube was recovered from the outside of the hydrophilized (PVA-treated) PTFE filter bag, and the obtained solution was defined as Through.

As a wash buffer, 4 mL of PBS (manufactured by Takara Bio Inc., model number: T900) was added to the outside of the hydrophilized (PVA-treated) PTFE filter bag, and the HT carrier particles were cleaned by sucking and discharging 2800 µL of the solution at a speed of speed 1 for 5 minutes according to a custom mode of the automatic pipettor. Thereafter, the liquid present in the tube was recovered from the outside of the hydrophilized (PVA-treated) PTFE filter bag, and this solution was defined as Wash.

0.4 mL of Lysis buffer-1 (250 mM EDTA aqueous solution, pH8.0, manufactured by NIPPON GENE CO., LTD., model number: 311-90075 diluted with water) was added into the hydrophilized (PVA-treated) PTFE filter bag, and the liquid that came out to the outside was added again into the hydrophilized (PVA-treated) PTFE filter bag. This operation was repeated 5 times, and bovine serum albumin labeled with a Cy3 fluorescent dye adsorbed to the HT carrier particles was released from the HT carrier particles. Thereafter, the liquid present in the tube was recovered from the outside of the hydrophilized (PVA-treated) PTFE filter bag and the obtained solution was defined as Elute-1. Thereafter, 0.4 mL of Lysis buffer-2 (0.1% (w/v) SDS aqueous solution) was further added into the hydrophilized (PVA-treated) PTFE filter bag, and the liquid that came out to the outside was added again into the hydrophilized (PVA-treated) PTFE filter bag. This operation was repeated 5 times, and bovine serum albumin labeled with a Cy3 fluorescent dye adsorbed to the HT carrier particles was released from the HT carrier particles. Thereafter, the liquid present in the tube was recovered from the outside of the hydrophilized (PVA-treated) PTFE filter bag, and the obtained solution was defined as Elute-2.

Using 200 µL of each of the liquids Input, Through, Wash, Elute-1, and Elute-2, the fluorescence value in each liquid under the conditions of excitation light of 470 nm and fluorescence of 510 to 580 nm was measured with a Qubit4 fluorometer (manufactured by Thermo Fisher Scientific Inc.).

The results are summarized in Table 1.

**[Table 1]**

| Fraction | Fluorescence value |
|---|---|
| Input | 1445 |
| Through | 315 |
| Wash | 70 |
| Elute-1 | 6274 |
| Elute-2 | 2328 |

The binding rate and release rate to the carrier were calculated from the following Formulas (1) and (2), respectively, and are shown in Table 2. Binding rate to carrier (%) = 100 - (Fluorescence value of Through?Fluorescence value of Input × 100) Release rate (%) = Fluorescence value of Elute × Liquid amount of Lysis buffer/{ (Fluorescence value of Input - Fluorescence value of Through) × Liquid amount of Input) × 100

The release rate calculated using only Elute-1 was defined as a release rate (1), and the release rate calculated using both Elute-1 and Elute-2 was defined as a release rate (1 + 2).

**[Table 2]**

| | |
|---|---|
| Binding rate to carrier (%) | 78.2 |
| Release rate (1) (%) | 55.5 |
| Release rate (1+2) (%) | 76.1 |

From the above results, it was possible to produce the purified liquid (Elute-1, Elute-2, and mixed liquid thereof) having an increased purity of the bovine serum albumin which is the substance α from the bovine serum albumin solution (sample solution containing the substance α).

### [Example 2]

A PVDF porous membrane (manufactured by Merck KGaA, Durapore SVLP09050, pore diameter 5 µm) hydrophilized was formed into a tubular shape (cylindrical shape having a bottom surface and an opening in the upper portion) having an inner diameter of 11 mm and a height of 95 mm using a thermal sealer (manufactured by AS ONE CORPORATION, AS-200) to prepare a PVDF filter bag 1.

A spiral-shaped spacer made of stainless steel (manufactured by DAIDOHANT.CO.,LTD., yarn count: 30) was inserted into the PVDF filter bag 1 in order to maintain the shape. The PVDF filter bag 1 with a spacer was put into a 14 mL tube (manufactured by FALCON, model number: 352059), and the upper portion of the PVDF filter bag 1 was fixed to the upper portion of the tube with a clip. At this time, the PVDF filter bag 1 was fixed to have an opening in the upper portion. That is, a container (tube) having a first chamber having an opening in the upper portion (the PVDF filter bag 1 with a spacer having an opening in the upper portion), and a second chamber partitioned from the first chamber by the PVDF filter bag 1 and having an opening in the upper portion (a part in a tube having an opening in the upper portion and outside the PVDF filter bag 1) was obtained.

Thereafter, into the PVDF filter bag 1, 50 µL of HT carrier particles (Bio-Gel HT Hydroxyapatite manufactured by Bio-Rad Laboratories, Inc., model number: 130-0150) was added with a micropipette.

The cultured influenza A virus (10^{9.0} TCID₅₀/mL) was diluted 500,000 times with PBS (manufactured by FUJIFILM Corporation, model number: 045-29795) to prepare an influenza A virus solution (2000 TCID₅₀/mL).

10 mL of this virus solution was added into the tube, that is, to the outside of the PVDF filter bag 1, with a 10 mL measuring pipette (manufactured by FALCON, model number: 357551). This virus solution was defined as Input.

A pipette tip of an automatic pipettor (manufactured by Gilson, Inc., model number: P10mLM, for 10 mL) was put into the tube, that is, to the outside of the PVDF filter bag 1, perpendicularly to the liquid surface, and the automatic pipettor was disposed to allow suction and discharge of the solution. Note that the arrangement of the container (tube) and the automatic pipettor is a representation of an automation apparatus including only pipetting means for sucking and discharging a solution and means for moving the pipetting means up and down and back and forth.

With the custom mode of the automatic pipettor, 6.5 mL of solution was sucked once at speed 2, followed by a 2-second wait, and then 2 mL of solution was sucked once at speed 2, followed by a 2-second wait. Next, 8.5 mL of solution was discharged once at speed 3, followed by a 5-second wait. This movement was repeated, and the HT carrier particles were sucked and discharged for 1 hour to adsorb the influenza A virus to the HT carrier particles.

After the solution was sucked and discharged for 1 hour, the liquid present in the tube was recovered from the outside of the PVDF filter bag 1, and the obtained solution was defined as Through.

The subsequent steps of adding and recovering the liquid were performed with a 1 mL measuring pipette (manufactured by FALCON, model number: 4485).

By adding 1 mL of Lysis buffer (5 M guanidine thiocyanate, 100mM EDTA (pH8.0), 100 mM Tris-HCl (pH6.8), 3% (w/v) Triton X-100, 1% (v/v) 2-mercaptoethanol into the PVDF filter bag 1, the influenza A virus adsorbed to the HT carrier particles was destroyed, and nucleic acids containing viral RNA were released from the HT carrier particles. Thereafter, the liquid present in the tube was recovered from the outside of the PVDF filter bag 1. 100 µL of this recovered liquid was subjected to an automatic nucleic acid extraction apparatus (manufactured by Precision System Science Co., Ltd., magLEAD 12gC), nucleic acids containing viral RNA were extracted using a nucleic acid extraction reagent (manufactured by Precision System Science Co., Ltd., MagDEA Dx SV), and the obtained solution was defined as Elute.

Quantitative PCR for M gene of influenza A virus, which is an example of a gene unique to influenza A virus, was performed using Input, Through, and Elute as PCR samples. Quantitative PCR was performed using primers and probes (MP-39-67For, MP-183-153Rev, and MP-96-75ProbeAs) for detecting the M gene of influenza A virus shown by Nakauchi et al. (Journal of Virological Methods, 2011, One-step real-time reverse transcription-PCR assays for detecting and subtyping pandemic influenza A/H1N1 2009, seasonal influenza A/H1N1, and seasonal influenza A/H3N2 viruses), a commercially available quantitative PCR kit (manufactured by TOYOBO Co., Ltd., THUNDERBIRD Probe One-step qRT-PCR Kit), and a quantitative PCR apparatus (7500 Fast Real-Time PCR System manufactured by Applied Biosystems). The experiment was carried out with n = 2.

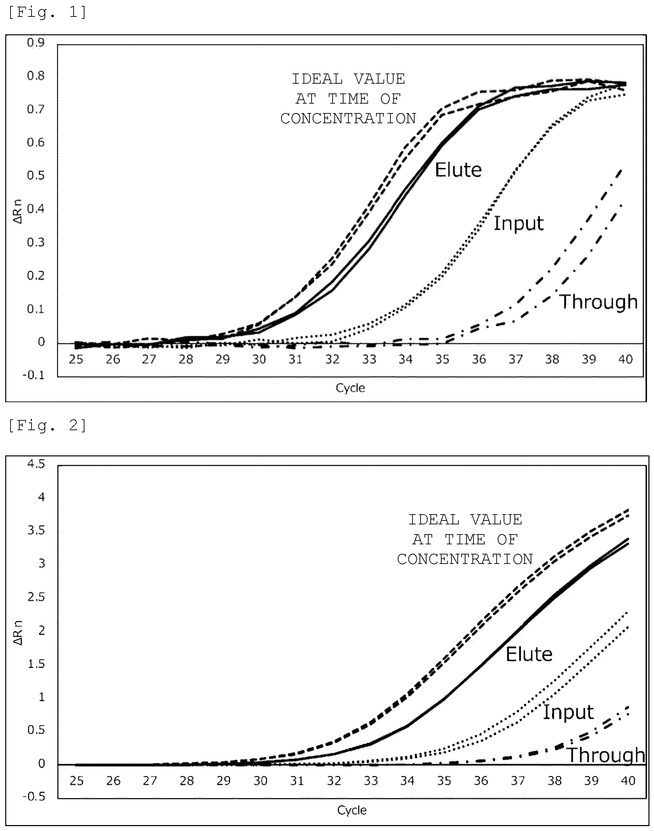

The results (amplification curves in quantitative PCR) are shown in Fig. 1. The vertical axis of the graph represents ΔRn (intensity of a fluorescence signal generated under a predetermined PCR condition), and the horizontal axis represents the number of cycles in quantitative PCR.

In addition to ΔRn when Input, Through, and Elute were used as PCR samples, ideal values at the time of concentration were shown. The ideal value at the time of concentration was calculated by preparing an influenza A virus solution (that is, 20,000 TCID₅₀/mL) which was 10 times thicker than Input, extracting nucleic acids containing viral RNA from the solution in the same manner as described above, and using the obtained solution as a PCR sample.

In Elute, ΔRn increased with fewer cycles than in Input, and it was revealed that a large amount of M gene was contained. That is, it was possible to produce a purified liquid (Elute) having an increased purity of viral RNA which is the substance α from the virus solution (sample solution containing the substance β). For example, at ΔRn = 0.4, the cycle number of Elute was about 3 smaller than that of Input, and thus it was revealed that the purity of viral RNA was increased by about 10 times. In addition, in Elute, the amplification curve was close to the ideal value at the time of concentration.

### [Example 3]

A PVDF porous membrane (manufactured by Merck KGaA, Durapore SVLP09050, pore diameter 5 µm) hydrophilized was formed into a tubular shape (cylindrical shape having a bottom surface and an opening in the upper portion) having an inner diameter of 6.4 mm and a height of 40 mm using a thermal sealer (manufactured by AS ONE CORPORATION, AS-200) to prepare a PVDF filter bag 2.

A hand made stainless steel spiral spacer (manufactured by DAIDOHANT.CO.,LTD., yarn count: 30, diameter: 5 mm, length: 4 cm) was inserted into the PVDF filter bag 2 in order to maintain the shape. The PVDF filter bag 2 with a spacer was put into a 2 mL tube (manufactured by Eppendorf SE, model number: 022431102), and the upper portion of the PVDF filter bag 2 was fixed to the upper portion of the tube with a clip. In addition, at this time, the PVDF filter bag 2 was fixed to have an opening in the upper portion. That is, a container (tube) having a first chamber having an opening in the upper portion (the PVDF filter bag 2 with a spacer having an opening in the upper portion), and a second chamber partitioned from the first chamber by the PVDF filter bag 2 and having an opening in the upper portion (a part in a tube having an opening in the upper portion and outside the PVDF filter bag 2) was obtained.

Thereafter, into the PVDF filter bag 2, 50 µL of HT carrier particles (Bio-Gel HT Hydroxyapatite manufactured by Bio-Rad Laboratories, Inc., model number: 130-0150) was added with a micropipette.

Lyophilized human serum-derived exosomes (manufactured by HansaBioMed Life Sciences Ltd, HBM-PES-30) was dissolved in water to prepare 1 µg/µL of an exosome solution. This exosome solution was diluted with a 1% (v/v) EV-Save (manufactured by FUJIFILM Corporation, model number: 058-09261)/PBS solution to prepare an exosome solution of 5 µg/mL (1.67 × 10⁵ pieces/mL).

1 mL of this exosome solution was added in a tube and outside the PVDF filter bag 2 with a micropipette. This exosome solution was defined as Input.

A pipette tip of an automatic pipettor (Thermo Fisher Scientific Inc., model number: E1-CLIP TIP, for 1 mL) was put into the tube and outside the PVDF filter bag 2 perpendicularly to the liquid surface, and the automatic pipettor was disposed to allow suction and discharge of the solution. Note that the arrangement of the container (tube) and the automatic pipettor is a representation of an automation apparatus including only pipetting means for sucking and discharging a solution and means for moving the pipetting means up and down and back and forth.

By sucking and discharging 0.7 mL of the solution at speed 1 for 1 hour according to the programs mode of the automatic pipettor, the human serum-derived exosomes were adsorbed to the HT carrier particles.

Subsequent steps of adding and recovering the liquid were performed with a micropipette.

After the solution was sucked and discharged for 1 hour, the liquid present in the tube was recovered from the outside of the PVDF filter bag 2, and the obtained solution was defined as Through.

500 µL of QIAzol Lysis Reagent contained in the miRNeasy kit (manufactured by QIAGEN) was added into the PVDF filter bag 2, and nucleic acids were released from the HT carrier particles. Thereafter, the liquid present in the tube was recovered from the outside of the PVDF filter bag 2, and the step of adding QIAzol Lysis Reagent into the PVDF filter bag 2 again was repeated 5 times, and nucleic acids were released from the HT carrier particles. Thereafter, the liquid present in the tube was recovered from the outside of the PVDF filter bag 2, and a microRNA fraction was obtained according to the protocol of miRNeasy kit. The resulting solution was defined as Elute.

Using Input, Through, and Elute as PCR samples, a quantitative PCR apparatus (manufactured by Applied Biosystems, 7500 Fast Real-Time PCR System) was used according to the product protocol using TaqMan MicroRNA Reverse Transcription Kit (manufactured by Invitrogen) and TaqMan microRNA Assay (has-mir-181a) (manufactured by Invitrogen), respectively, to perform quantitative PCR for miR181a, which is one type of microRNA. The experiment was carried out with n = 2.

The results (amplification curves in quantitative PCR) are shown in Fig. 2. The vertical axis of the graph represents ΔRn (intensity of a fluorescence signal generated under a predetermined PCR condition), and the number of cycles in quantitative PCR.

In addition to ΔRn when Input, Through, and Elute were used as PCR samples, ideal values at the time of concentration were shown. The ideal value at the time of concentration was calculated by preparing an exosome solution (that is, 50 µg/mL) which was 10 times thicker than Input, extracting a microRNA fraction from the solution in the same manner as described above, and using the obtained solution as a PCR sample.

In Elute, ΔRn increased with fewer cycles than in Input, and it was revealed that a large amount of miR181a was contained. That is, it was possible to produce a purified liquid (Elute) having an increased purity of microRNA which is the substance α from the exosome solution (sample solution containing the substance β). For example, at ΔRn = 1.5, the cycle number of Elute was about 3 smaller than that of Input, and thus it was revealed that the purity of microRNA was increased by about 10 times. In addition, in Elute, the amplification curve was close to the ideal value at the time of concentration.

### [Examples 4 and 5]

### (Preparation of Hydrophilized (Hydroxyacrylate-treated) PTFE Filter Bag)

A nonwoven fabric (weight: 16 g/m², 300 mm × 20 mm, average pore diameter: 1 µm) composed of ultrafine PTFE fibers was immersed in a 99.7% isopropyl alcohol (IPA) solution (manufactured by FUJIFILM Wako Pure Chemical Corporation) for 1 minute at room temperature (25°C).

Subsequently, the PTFE nonwoven fabric taken out from the IPA solution was immersed at 65°C for 1 hour in an aqueous solution in which 7 g of hydroxypropyl methacrylate (mixture of 2-hydroxypropyl ester and 2-hydroxy-1-methylethyl ester) (HPA) (manufactured by Tokyo Chemical Industry Co., Ltd., product code: M0512), 0.7 g of tetraethylene glycol diacrylate (TEGDA) (manufactured by Tokyo Chemical Industry Co., Ltd., product code: T1569), and 1.3 g of ammonium persulfate (AmPS) (manufactured by Tokyo Chemical Industry Co., Ltd., product code: A2098) were dissolved in 100 g of pure water.

Thereafter, the PTFE nonwoven fabric taken out from the aqueous solution was put into pure water and boiled at 85°C for 6 hours to dissolve unreacted IPA, HPA, TEGDA, and AmPS.

After boiling, the PTFE nonwoven fabric taken out from the liquid was naturally dried, and the nonwoven fabric was formed into a cylindrical shape (cylindrical shape having a bottom surface and an opening in the upper portion) having an inner diameter of 6 mm and a height of 90 mm using a double-sided tape (manufactured by Nichiban Co., Ltd., NICETACK strong type, #NW-K10) to prepare a hydrophilized (hydroxyacrylate-treated) PTFE filter bag.

### (Preparation of Hydrophilized (PVA-MPC-treated) PTFE Filter Bag)

A nonwoven fabric (weight: 16 g/m², 300 mm × 20 mm, average pore diameter: 1 µm) composed of ultrafine PTFE fibers was immersed in a 99.7% isopropyl alcohol (IPA) solution (manufactured by FUJIFILM Wako Pure Chemical Corporation) for 1 minute at room temperature (25°C).

Next, the PTFE nonwoven fabric taken out from the IPA solution was immersed in 500 mL of a polyvinyl alcohol (PVA) (manufactured by FUJIFILM Wako Pure Chemical Corporation, 160-11485, polymerization degree: 1500, saponification degree: 98%) aqueous solution adjusted to a concentration of 0.1 mass% at room temperature for 30 minutes.

Thereafter, the PTFE filter taken out from the PVA aqueous solution was immersed in a mixed liquid obtained by mixing 500 mL of a 5 mass% glutaraldehyde solution (a solution prepared by diluting a 25% glutaraldehyde solution manufactured by FUJIFILM Wako Pure Chemical Corporation with pure water to adjust a concentration to 5 mass%) and 5 mL of a 36% aqueous hydrochloric acid solution (manufactured by FUJIFILM Wako Pure Chemical Corporation) at room temperature for 30 minutes.

Next, the PTFE nonwoven fabric taken out from the mixed liquid was put into pure water to dissolve unreacted IPA, PVA, and glutaraldehyde.

Thereafter, the PTFE nonwoven fabric taken out from the liquid was naturally dried to obtain a hydrophilized (PVA-treated) PTFE filter.

Furthermore, the obtained hydrophilized (PVA-treated) PTFE filter was immersed in an ethanol solution (manufactured by Intelligent Surfaces, Inc., MP011L1) of 2-methacryloyloxyethyl phosphorylcholine (MPC) polymer at room temperature (25°C) for 30 minutes.

Thereafter, the filter taken out from the ethanol solution was naturally dried, then cleaned with pure water, and the naturally dried nonwoven fabric was formed into a tubular shape (cylindrical shape having a bottom surface and an opening in the upper portion) having an inner diameter of 6 mm and a height of 90 mm using a double-sided tape (manufactured by Nichiban Co., Ltd., NICETACK strong type, #NW-K10) to prepare a hydrophilized (PVA-MPC-treated) PTFE filter bag.

Spacers made of stainless steel (manufactured by DAIDOHANT.CO.,LTD., yarn count: 30) and having a spiral shape were respectively inserted into a hydrophilized (hydroxyacrylate-treated) PTFE filter bag (Example 4) and a hydrophilized (PVA-MPC-treated) PTFE filter bag (Example 5) in order to maintain the shape. A hydrophilized (hydroxyacrylate-treated) PTFE filter bag and a hydrophilized (PVA-MPC-treated) PTFE filter bag with a spacer were each put into a 14 mL tube (manufactured by FALCON, model number: 352059), and the upper portions of the filter bags were fixed to the upper portion of the tube with a clip. At this time, the filter bags were fixed to have an opening in the upper portions. Thereafter, into each of the filter bags, 100 µL of 50% aqueous solution (vol/vol) of HT carrier particles (Bio-Gel HT Hydroxyapatite manufactured by Bio-Rad Laboratories, Inc., model number: 130-0150) was added with a micropipette.

The cultured influenza A virus (109.0 TCID50/mL) was diluted 500,000 times with D-PBS (manufactured by FUJIFILM Corporation, model number: 045-29795) to prepare an influenza A virus solution (2000 TCID50/mL).

10 mL of this virus solution was added into the tube, that is, to the outside of the filter bag, with a 10 mL measuring pipette (manufactured by FALCON, model number: 357551). This virus solution was defined as Input.

A pipette tip of an automatic pipettor (manufactured by Gilson, Inc., model number: P10mLM, for 10 mL) was put into the tube and the outside of the filter bag perpendicularly to the liquid surface, and the automatic pipettor was disposed to allow suction and discharge of the solution. With the custom mode of the automatic pipettor, 6.5 mL of solution was sucked once at speed 2, followed by a 2-second wait, and then 2 mL of solution was sucked once at speed 2, followed by a 2-second wait. Next, 8.5 mL of solution was discharged once at speed 3, followed by a 5-second wait. This movement was repeated, and the HT carrier particles were sucked and discharged for 1 hour to adsorb the influenza A virus to the HT carrier particles.

After the solution was sucked and discharged for 1 hour, the liquid present in the tube was recovered from the outside of the filter bag, and the obtained solution was defined as Through.

The subsequent steps of adding and recovering the liquid were performed with a 1 mL measuring pipette (manufactured by FALCON, model number: 4485).

Lysis buffer (5 M guanidine thiocyanate, 100mM EDTA (pH8.0), 100 mM Tris-HCl (pH6.8), 3% (w/v) Triton X-100, 1% (v/v) 2-mercaptoethanol 1 mL was added into the filter bag, after which the liquid present in the tube was recovered from the outside of the filter bag and again added into the filter bag. This operation was repeated for 5 minutes to destroy the influenza A virus adsorbed to the HT carrier particles and release the nucleic acids containing the viral RNA from the HT carrier particles. 100 µL of this recovered liquid was subjected to an automatic nucleic acid extraction apparatus (manufactured by Precision System Science Co., Ltd., magLEAD 12gC), nucleic acids containing viral RNA were extracted using a nucleic acid extraction reagent (manufactured by Precision System Science Co., Ltd., MagDEA Dx SV), and the obtained solution was defined as Elute.

Quantitative PCR for M gene of influenza A virus, which is an example of a gene unique to influenza A virus, was performed using Input, Through, and Elute as PCR samples. Quantitative PCR was performed using primers and probes (MP-39-67For, MP-183-153Rev, and MP-96-75ProbeAs) for detecting the M gene of influenza A virus shown by Nakauchi et al., a commercially available quantitative PCR kit ( manufactured by TOYOBO Co., Ltd., THUNDERBIRD Probe One-step qRT-PCR Kit), and a quantitative PCR apparatus (7500 Fast Real-Time PCR System manufactured by Applied Biosystems). The experiment was carried out with n = 2.

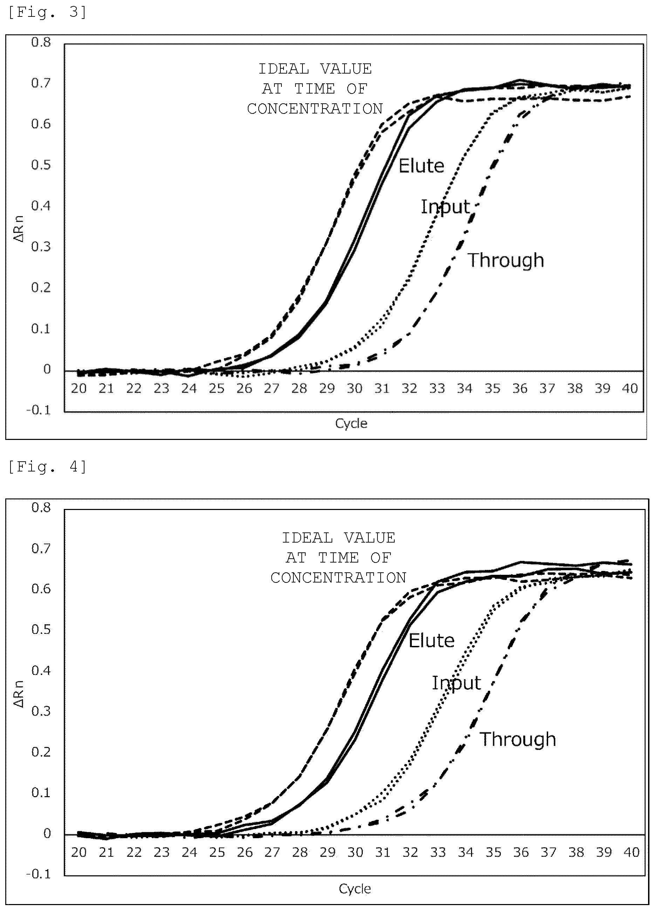

The results (amplification curves in quantitative PCR) are shown in Fig. 3 (hydrophilized (hydroxyacrylate-treated) PTFE filter bag) and Fig. 4 (hydrophilized (PVA-MPC-treated) PTFE filter bag). The vertical axis of the graph represents ΔRn (intensity of a fluorescence signal generated under a predetermined PCR condition), and the horizontal axis represents the number of cycles in quantitative PCR.

In addition to ΔRn when Input, Through, and Elute were used as PCR samples, ideal values at the time of concentration were shown. The ideal value at the time of concentration was calculated by preparing an influenza A virus solution (that is, 20,000 TCID50/mL) which was 10 times thicker than Input, extracting nucleic acids containing viral RNA from the solution in the same manner as described above, and using the obtained solution as a PCR sample.

In Elute, ΔRn increased with fewer cycles than in Input, and it was revealed that a large amount of M gene was contained. That is, it was possible to produce a purified liquid (Elute) having an increased purity of viral RNA which is the substance α from the virus solution (sample solution containing the substance β). For example, at ΔRn = 0.4, the cycle number of Elute was about 3 smaller than that of Input, and thus it was revealed that the purity of viral RNA was increased by about 10 times. In addition, in Elute, the amplification curve was close to the ideal value at the time of concentration.

### [Example 6]

A PTFE nonwoven fabric hydrophilized (hydroxyacrylate-treated) in the same manner as in Example 4 was formed into a tubular shape (cylindrical shape having a bottom surface and an opening in the upper portion) having an inner diameter of 16 mm and a height of 40 mm using a double-sided tape (manufactured by Nichiban Co., Ltd., NICETACK strong type, #NW-K10) to prepare a hydrophilized (hydroxyacrylate-treated) PTFE filter bag. An experiment to concentrate exosomes was performed using this hydrophilized (hydroxyacrylate-treated) PTFE filter bag.

A hand made stainless steel spiral spacer (manufactured by DAIDOHANT.CO.,LTD., yarn count: 30, diameter: 5 mm, length: 40 mm) was inserted into the filter bag in order to maintain the shape. The filter bag with a spacer was put into a 2 mL tube (manufactured by Eppendorf SE, model number: 022431102), and the upper portion of the filter bag was fixed to the upper portion of the tube with a clip. At this time, the filter bag was fixed to have an opening in the upper portion. That is, a container (tube) having a first chamber having an opening in the upper portion (a hydrophilized (hydroxyacrylate-treated) PTFE filter bag with a spacer having an opening in the upper portion), and a second chamber partitioned from the first chamber by a hydrophilized (hydroxyacrylate-treated) PTFE filter bag and having an opening in the upper portion (a part in a tube having an opening in the upper portion and outside the hydrophilized (hydroxyacrylate-treated) PTFE filter bag) was obtained.

Thereafter, 100 µL of exosome capture resin (manufactured by FUJIFILM Corporation, PS Capture Exosome Isolation Resin Kit, #290-80301, average particle diameter: about 34 µm) was added into the filter bag with a micropipette.

Lyophilized human urine-derived exosome (manufactured by HansaBioMed Life Sciences Ltd, HBM-PEU-100/2) derived from a healthy person was dissolved in water to prepare 1 µg/µL of an exosome solution. This exosome solution was diluted with a 0.1% (v/v) bovine serum albumin (BSA)/PBS solution and Binding Enhancer(manufactured by FUJIFILM Corporation, PS Capture Exosome Isolation Resin Kit, #290-80301) to prepare an exosome solution of 5 µg/mL (4.7 × 10⁹ pieces/mL). Since the exosome solution contains bovine serum albumin as a contaminating protein, the exosome solution imitates not only exosomes but also, for example, biological samples (for example, blood or urine) containing contaminating proteins.

1 mL of this exosome solution was added into a tube, that is, to the outside of the filter bag with a micropipette. This exosome solution was defined as Input.

A pipette tip of an automatic pipettor (Thermo Fisher Scientific Inc., model number: E1-CLIP TIP, for 1 mL) was put into the tube, that is, to the outside of the filter bag, perpendicularly to the liquid surface, and the automatic pipettor was disposed to allow suction and discharge of the solution. Note that the arrangement of the container (tube) and the automatic pipettor is a representation of an automation apparatus including only pipetting means for sucking and discharging a solution and means for moving the pipetting means up and down and back and forth.

The human urine-derived exosome was adsorbed to the exosome capture resin by sucking and discharging 0.7 mL of the solution at speed 1 for 1 hour according to the programs mode of the automatic pipettor.

Subsequent steps of adding and recovering the liquid were performed with a micropipette.

After the solution was sucked and discharged for 1 hour, the liquid present in the tube was recovered from the outside of the filter bag, and the obtained solution was defined as Through.

500 µL of QIAzol Lysis Reagent contained in the miRNeasy kit (manufactured by QIAGEN) was added into the filter bag, and nucleic acids were released from the exosome capture resin. Thereafter, the step of recovering the liquid present in the tube from the outside of the filter bag and adding the liquid into the filter bag again was repeated for 5 minutes, and the nucleic acids were released from the exosome capture resin. Thereafter, the liquid present in the tube was recovered from the outside of the filter bag, and a microRNA fraction was obtained according to the protocol of miRNeasy kit. The resulting solution was defined as Elute.

Using Input, Through, and Elute as PCR samples, a quantitative PCR apparatus (manufactured by Applied Biosystems, 7500 Fast Real-Time PCR System) was used according to the product protocol using TaqMan MicroRNA Reverse Transcription Kit (manufactured by Invitrogen) and TaqMan microRNA Assay (has-mir-181a) (manufactured by Invitrogen), respectively, to perform quantitative PCR for miR181a, which is one type of microRNA. The experiment was carried out with n = 2.

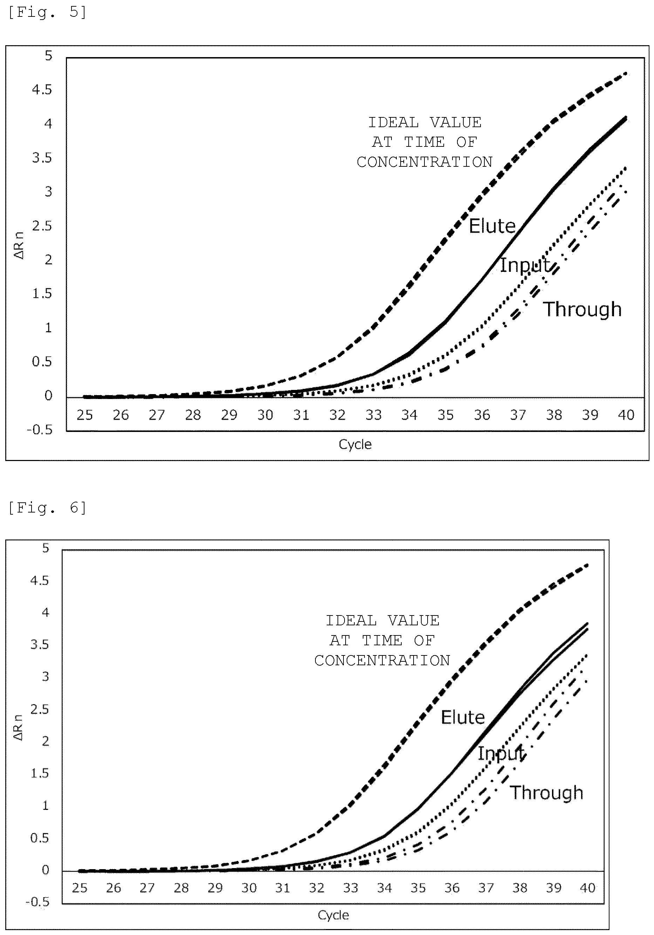

The results (amplification curves in quantitative PCR) are shown in Fig. 5. The vertical axis of the graph represents ΔRn (intensity of a fluorescence signal generated under a predetermined PCR condition), and the number of cycles in quantitative PCR.

In addition to ΔRn when Input, Through, and Elute were used as PCR samples, ideal values at the time of concentration were shown. The ideal value at the time of concentration was calculated by preparing an exosome solution (that is, 50 µg/mL) which was 10 times thicker than Input, extracting a microRNA fraction from the solution in the same manner as described above, and using the obtained solution as a PCR sample.

In Elute, ΔRn increased with fewer cycles than in Input, and it was revealed that a large amount of miR181a was contained. That is, it was possible to produce a purified liquid (Elute) having an increased purity of microRNA which is the substance α from the exosome solution (sample solution containing the substance β). For example, at ΔRn = 1.5, the cycle number of Elute was about 1.7 smaller than that of Input, and thus it was revealed that the purity of microRNA was increased by about 3.25 times.

### [Examples 7 and 8]

A concentration experiment was carried out in the same manner as in Example 6, except that the same hydrophilized (PVA-MPC-treated) PTFE filter bag as in Example 5 was used, and the amount of Binding Enhancer added was 1 time and 10 times. The case where the amount of Binding Enhancer was increased by 1 was defined as Example 7, and the case where the amount of Binding Enhancer was increased by 10 was defined as Example 8.

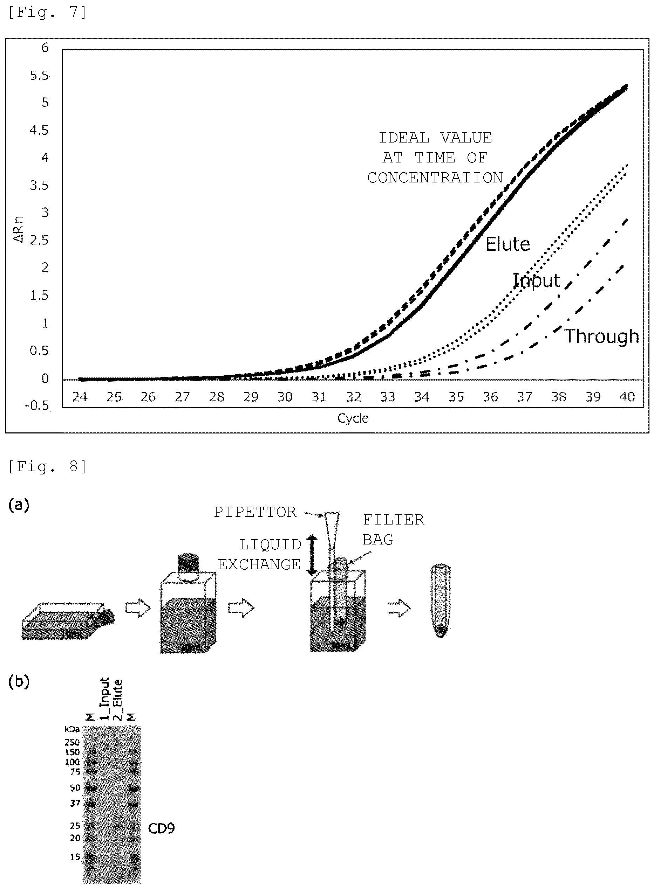

The results are shown in Fig. 6 (Example 7) and Fig. 7 (Example 8). As the amount of Binding Enhancer added increased, the amount of exosome concentrated increased, and an amplification curve closer to an ideal value was shown in quantitative PCR.

### [Example 9]

Capan2, which is a cultured cell derived from pancreatic cancer, was used as an exosome supply source cell to conduct an experiment to concentrate exosomes from cell culture supernatant. Conventionally, it has been known that culture supernatants such as adipocytes include exosomes, and, for example, adipocytes have been widely used as source cells for exosomes. However, Capan2, which is a pancreatic cancer-derived culture cell, was used as an exosome supply source cell this time.

The outline of the experiment is shown in Fig. 8(a).

For culturing Capan2 cells, a CELLSTAR flask manufactured by Greiner Bio-One International GmbH, 25cm² (690170) and a serum-containing medium (D-MEM medium (manufactured by FUJIFILM Corporation, #044-29765), 10% Fetal Bovine Serum(manufactured by Thermo Fisher Scientific Inc. (Gibco), #10270106), 1% Penicillin-Streptomycin(manufactured by Thermo Fisher Scientific Inc. (Gibco), #15140122) were used.

Fetal Bovine Serum used for normal cell culture includes bovine-derived exosomes. In order to prevent mixing of the bovine-derived exosomes into the cell culture supernatant, Capan2 cells were cultured in a serum-free medium (D-MEM medium (manufactured by FUJIFILM Corporation, #044-29765), 1% Penicillin-Streptomycin (manufactured by Thermo Fisher Scientific Inc. (Gibco), #15140122) from the middle of the culture period. Specifically, the culture supernatant was removed from a flask of Capan2 cells grown to a confluent state in a serum-containing medium, and subsequently a cell cleaning operation (an operation of adding 10 mL of serum-free medium to the flask, cleaning the cells, and then immediately removing the medium) was performed 5 times. Subsequently, 10 mL of a serum-free medium was added to the flask and cultured at 37°C and a 5% CO₂ concentration for 48 hours.

After 48 hours of culture, the flask was erected and 20 mL of serum-free medium was added to make the total amount 30 mL. This was defined as Input before concentration (Fig. 8(b)).

A nonwoven fabric made of ultrafine PTFE fibers hydrophilized with PVA and MPC in the same manner as in Example 5 was formed into a cylindrical shape (cylindrical shape having a bottom surface and an opening in the upper portion) with an inner diameter of 20 mm and a height of 90 mm using a double-sided tape (manufactured by Nichiban Co., Ltd., NICETACK strong type, #NW-K10) to prepare a hydrophilized (PVA-MPC-treated) PTFE filter bag, and a spiral spacer made of stainless steel (manufactured by DAIDOHANT.CO.,LTD., yarn count: 30) was inserted in order to maintain the shape. Into a hydrophilized (PVA-MPC-treated) PTFE filter bag with a spacer, 60 µL of exosome capture resin (manufactured by FUJIFILM Corporation, PS Capture Exosome Isolation Resin Kit, #290-80301) was added with a micropipette, and the filter bag was fixed in a flask with a clip such that the lower end 1 cm of the filter bag was immersed in the medium.

A pipette tip of an automatic pipettor (manufactured by Gilson, Inc., model number: P10mLM, for 10 mL) was put into the flask, that is, to the outside of the filter bag, perpendicularly to the liquid surface, and the automatic pipettor was disposed to allow suction and discharge of the solution. With the custom mode of the automatic pipettor, 8 mL of solution was sucked once at a speed 3, and then 8 mL of solution was discharged once at a speed 3, followed by a 10-second wait. This movement was repeated, and the exosomes in the culture supernatant were adsorbed to the exosome capture resin by suction and discharge for 1 hour.

After the adsorption operation, the filter bag was taken out from the flask and put into a tube (manufactured by FALCON, model number: 352059) having a volume of 14 mL, and the upper portion of the filter bag was fixed to the upper portion of the tube with a clip. At this time, the filter bag was fixed to have an opening in the upper portion. The subsequent steps of adding and recovering the liquid were performed with a 1 mL measuring pipette (manufactured by FALCON, model number: 4485). An operation of adding 1 mL of a cleaning fluid (manufactured by FUJIFILM Corporation, PS Capture Exosome Isolation Resin Kit, #290-80301) into the filter bag and recovering the mixture from the outside of the filter bag was performed 3 times to clean a substance non-specifically binding to the exosome capture resin. Subsequently, 100 µL of an eluate (manufactured by FUJIFILM Corporation, PS Capture Exosome Isolation Resin Kit, #290-80301) was added into the filter bag and left to stand at room temperature for 10 minutes, and then the eluate was recovered from the outside of the filter bag. This was defined as Elute.

The confirmation of the exosome concentrated from the culture supernatant of Capan2 cells was performed by Western blot of CD9, which is a marker of the exosome. 15 µL of the eluate was mixed with a sample buffer not containing a reducing agent (manufactured by FUJIFILM Corporation, #198-13282), treated at 100°C for 5 minutes, and developed by SDS-PAGE.

As a comparative control, 15 µL of an unconcentrated Input sample was subjected to the same treatment.

As a gel for SDS-PAGE, SuperSep (TM) Ace, 10-20% (manufactured by FUJIFILM Corporation, #191-15031) was used. After electrophoresis, the developed sample was transferred from the gel to a PVDF membrane (Trans-Blot Turbo Mini 0.2 µm PVDF Transfer Packs, manufactured by Bio-Rad Laboratories, Inc., #1704156), and the PVDF membrane was blocked for 1 hour at room temperature using PVDF Blocking Reagent for Can Get Signal (manufactured by TOYOBO Co., Ltd., #NYPBR01). As the detection antibody, anti-Human CD9 (COSMO BIO, #SHI-EXO-M01) as a primary antibody and anti-Mouse IgG-HRP(GE, #NA931) as a secondary antibody were diluted 1000 times and 5000 times with Can Get Signal Solution (manufactured by TOYOBO Co., Ltd., #NKB-101), respectively, and used. The reactions were each performed at room temperature for 1 hour. Immobilon Forte (manufactured by MilliporeSigma, #WBLUF0100) was used as a detection reagent, and a signal was detected by LuminoGraph1 (manufactured by ATTO CORPORATION).

The results of the Western blot are shown in Fig. 8(b). In Input, a band reacting with the Anti-CD9 antibody could not be detected, whereas in the sample (Elute) concentrated using a hydrophilized (PVA-MPC-treated) PTFE filter bag and an exosome capture resin, a CD9 band was confirmed at around 25kDa. From this result, it was confirmed that the exosome can be concentrated from the cell culture supernatant.

From the above results, it was possible to produce a purified liquid (Elute) having an increased purity of the exosome, which is the substance α, from the cell culture supernatant containing the exosome (sample solution containing the substance α).

In recent years, the possibility of medical application, in which exosomes released by cells are purified from, for example, a cell culture supernatant, and the purified exosomes are administered into the body, has been studied. The method for producing a purified liquid according to an aspect of the present invention is also expected to be applied to such fields.

## Claims

1. A method for producing a purified liquid having an increased purity of a substance α from a sample solution containing a separation target substance (substance α) or a substance containing the substance α (substance β), the method comprising:
(1) step 1 and step 2-1 which will be described below; or
(2) step 1 and step 2-2 which will be described below (here, the substance α in the substance β is a protein, a nucleic acid, a lipid, or a saccharide),
in which a container (i) including a first chamber having an opening in an upper portion and a second chamber partitioned from the first chamber by a filter and having an opening in an upper portion is used,
step 1: a step of obtaining a container (ii) having a carrier to which the substance α or the substance β in the sample solution is adsorbed, in the first chamber,
step 2-1: a step of obtaining a purified liquid having an increased purity of the substance α by bringing a liquid capable of releasing the substance α from the carrier into contact with the carrier and releasing the substance α from the carrier, in the container (ii),
step 2-2: a step of obtaining a purified liquid having an increased purity of the substance α by bringing a liquid capable of releasing the substance α from the carrier into contact with the carrier and releasing the substance α from the carrier (here, the filter does not allow the carrier to pass therethrough).

2. The method for producing a purified liquid according to claim 1, wherein the step 2-1 or the step 2-2 is performed by a pipetting operation.

3. The method for producing a purified liquid according to claim 1 or 2, wherein the container (ii) is obtained by any one of the following steps a to d,
step a: a step of adsorbing the substance α or the substance β to the carrier by putting the sample solution into the second chamber and diffusing the substance α and the substance β in the sample solution, after a carrier capable of adsorbing the substance α or the substance β is put into the first chamber (here, the filter allows the substance α and the substance β to pass therethrough),
step b: a step of adsorbing the substance α or the substance β to the carrier by putting the sample solution into the first chamber and diffusing the substance α and the substance β in the sample solution, after a carrier capable of adsorbing the substance α or the substance β is put into the first chamber,
step c: a step of adsorbing a substance containing the substance α or the substance β to the carrier by providing the first chamber having a carrier capable of adsorbing the substance α or the substance β and the second chamber, and diffusing the substance α and the substance β in the sample solution, in a container (iii), after the sample solution is put into the container (iii) having an opening in the upper portion (here, the filter allows the substance α and the substance β to pass therethrough),
step d: a step of putting a carrier to which the substance α or the substance β is adsorbed into the first chamber after obtaining a carrier to which a substance containing the substance α or the substance β is adsorbed, by mixing the sample solution with a carrier capable of adsorbing the substance α or the substance β, and diffusing the substance α and the substance β in the sample solution.

4. The method for producing a purified liquid according to claim 3, wherein the method for diffusing the substance α and the substance β in the sample solution is a method for moving a liquid surface of the sample solution up and down.

5. The method for producing a purified liquid according to claim 3, wherein the method for diffusing the substance α and the substance β in the sample solution is performed by a pipetting operation.

6. The method for producing a purified liquid according to claim 3, further comprising: a step of removing a liquid present in the container (ii) after any one of the steps a to d, after any one of the steps a to d and before bringing the liquid capable of releasing the substance α from the carrier into contact with the carrier.

7. The method for producing a purified liquid according to claim 6, wherein the step of removing the liquid present in the container (ii) after any one of the steps a to d is performed by the pipetting operation.

8. The method for producing a purified liquid according to claim 6, wherein
the method for diffusing the substance α and the substance β in the sample solution,
the step of removing the liquid present in the container (ii) after any one of the steps a to d, and
any one of the step 2-1 and the step 2-2, are performed by the pipetting operation.

9. The method for producing a purified liquid according to claim 1 or 2, wherein the substance α is a nucleic acid or a protein.

10. The method for producing a purified liquid according to claim 9, wherein the substance β is a virus or an exosome.

11. The method for producing a purified liquid according to claim 1 or 2, wherein the substance α is an exosome.

12. A purification kit which is a purification kit for purifying a substance α from a sample solution containing a separation target substance (substance α) or a substance containing the substance α (substance β), the purification kit comprising:
a container; a carrier capable of adsorbing the substance α or the substance β; and a filter that does not allow the carrier to pass through, wherein
the filter is used to partition the container into a first chamber and a second chamber each having an opening in an upper portion, and the first chamber has the carrier.
